# EUROPEAN PATENT APPLICATION

(11) **EP 3 818 978 A1**
(43) Date of publication of application: **12.05.2021**
(21) Application number: 19208047.1
(22) Date of filing: 08.11.2019
(51) Int. Cl.: A61K 31/198, A61K 9/00, A61P 25/18

(54) **N-ACETYLCYSTEINE OR DERIVATIVES FOR TREATING COGNITIVE SYMPTOMS IN SCHIZOPHRENIA PATIENTS**

(71) Applicant: Stichting Katholieke Universiteit, 6525 XZ Nijmegen (NL)
(72) Inventor: Martens, Gerardus Julianus, 6522 HH Nijmegen (NL); Maas, Dorothea Adriana, 6525 AJ Nijmegen (NL)
(74) Representative: Nederlandsch Octrooibureau

(57) **Abstract**

The present invention concerns the field of Schizophrenia treatment. More in particular, the present invention is directed to pharmacological methods of treating and/or preventing one or more cognitive symptoms, in particular one or more prefrontal cortex (PFC) dependent cognitive symptoms. The present inventors have found that treatment of APO-SUS rats, showing SZ-relevant behavioural traits and neurobiological characteristics, with N-acetyl cysteine rescued interneuron hypomyelination and resulted in a significant improvement in cognitive inflexibility in the extra-dimensional set-shifting task. Hence, methods involving the administration of a pharmaceutical composition comprising N-acetyl cysteine or a pharmaceutically acceptable derivative are provided, as well as the pharmaceutical products that can be used in these methods.

SEQUENCE LISTING

<110>  Stichting Katholieke Universiteit

<120>  TREATMENT OF COGNITIVE SYMPTOMS IN SCHIZOPHRENIA PATIENTS

<130>  P6086569EP

<140>  EP 19208047.1
<141>  2019-11-08

<160>  38

<170>  PatentIn version 3.5

<210>  1
<211>  23
<212>  DNA
<213>  Artificial sequence

<220>
<223>  forward primer (beta-actin)

<400>  1
ccttcctggg tatggaatcc tgt                                              23


<210>  2
<211>  20
<212>  DNA
<213>  Artificial sequence

<220>
<223>  reverse primer (beta-actin)

<400>  2
tagagccacc aatccacaca                                                  20


<210>  3
<211>  20
<212>  DNA
<213>  Artificial sequence

<220>
<223>  forward primer (Ppia)

<400>  3
agcactgggg agaaaggatt                                                  20


<210>  4
<211>  20
<212>  DNA
<213>  Artificial sequence

<220>
<223>  reverse primer (Ppia)

<400>  4
agccactcag tcttggcagt                                                  20

<210>  5
<211>  20
<212>  DNA
<213>  Artificial sequence

<220>
<223>  forward primer (Gapdh)

<400>  5
gggtgtgaac cacgagaaat                                                    20


<210>  6
<211>  20
<212>  DNA
<213>  Artificial sequence

<220>
<223>  reverse primer (Gapdh)

<400>  6
actgtggtca tgagcccttc                                                    20


<210>  7
<211>  20
<212>  DNA
<213>  Artificial sequence

<220>
<223>  forward primer (Ywhaz)

<400>  7
ttgagcagaa gacggaaggt                                                    20


<210>  8
<211>  20
<212>  DNA
<213>  Artificial sequence

<220>
<223>  reverse primer (Ywhaz)

<400>  8
gaagcattgg ggatcaagaa                                                    20


<210>  9
<211>  20
<212>  DNA
<213>  Artificial sequence

<220>
<223>  forward primer (Cldn11)

<400>  9
cgcaaaatgg acgaactggg                                                    20


<210>  10
<211>  20
<212>  DNA

<213>  Artificial sequence

<220>
<223>  reverse primer (Cldn11)

<400>  10
tgcacgtaac cagggaggat                                                   20


<210>  11
<211>  21
<212>  DNA
<213>  Artificial sequence

<220>
<223>  forward primer (Mag)

<400>  11
aagccagacc atccaacctt c                                                 21


<210>  12
<211>  20
<212>  DNA
<213>  Artificial sequence

<220>
<223>  reverse primer (Mag)

<400>  12
ctcctgattc cgctccaagt                                                   20


<210>  13
<211>  25
<212>  DNA
<213>  Artificial sequence

<220>
<223>  forward primer (Mbp)

<400>  13
ccctactcca tcctcagact ttctt                                             25


<210>  14
<211>  19
<212>  DNA
<213>  Artificial sequence

<220>
<223>  reverse primer (Mbp)

<400>  14
tggcggtgtg cctgtctat                                                    19


<210>  15
<211>  21
<212>  DNA
<213>  Artificial sequence

<220>

<223>  forward primer (Mobp)

<400>  15
aatacctgca gggcaacaaa g                                                21


<210>  16
<211>  21
<212>  DNA
<213>  Artificial sequence

<220>
<223>  reverse primer (Mobp)

<400>  16
tctggttctt ggagagcctg g                                                21


<210>  17
<211>  20
<212>  DNA
<213>  Artificial sequence

<220>
<223>  forward primer (Mog)

<400>  17
cgccgtggag ttgaaagtag                                                  20


<210>  18
<211>  20
<212>  DNA
<213>  Artificial sequence

<220>
<223>  reverse primer (Mog)

<400>  18
gcacggagtt ttcctctagt                                                  20


<210>  19
<211>  20
<212>  DNA
<213>  Artificial sequence

<220>
<223>  forward primer (Opalin)

<400>  19
accctgatcc agcgaagaag                                                  20


<210>  20
<211>  23
<212>  DNA
<213>  Artificial sequence

<220>
<223>  reverse primer (Opalin)

<400>  20

tgactgccta ggattctcgg ata                                              23

<210>  21
<211>  20
<212>  DNA
<213>  Artificial sequence

<220>
<223>  forward primer (Plp1)

<400>  21
gggcctgagc gcaacggtaa                                                   20

<210>  22
<211>  21
<212>  DNA
<213>  Artificial sequence

<220>
<223>  reverse primer (Plp1)

<400>  22
caggcacagc agagcaggca a                                                 21

<210>  23
<211>  20
<212>  DNA
<213>  Artificial sequence

<220>
<223>  forward primer (Gsta4)

<400>  23
gccgccaagt acaacttgta                                                   20

<210>  24
<211>  26
<212>  DNA
<213>  Artificial sequence

<220>
<223>  reverse primer (Gsta4)

<400>  24
cactgctaaa gctaggctct cttctt                                            26

<210>  25
<211>  19
<212>  DNA
<213>  Artificial sequence

<220>
<223>  forward primer (Gstm4)

<400>  25
gcctaggccc ctggttttc                                                    19

<210>  26
<211>  22
<212>  DNA
<213>  Artificial sequence

<220>
<223>  reverse primer (Gstm4)

<400>  26
tcttcacagc agcacagcaa ct                                                22


<210>  27
<211>  24
<212>  DNA
<213>  Artificial sequence

<220>
<223>  forward primer (Prdx6)

<400>  27
tgactggaag aagggagaga gtgt                                              24


<210>  28
<211>  22
<212>  DNA
<213>  Artificial sequence

<220>
<223>  reverse primer (Prdx6)

<400>  28
atgggagctc tttggtgaag ac                                                22


<210>  29
<211>  20
<212>  DNA
<213>  Artificial sequence

<220>
<223>  forward primer (Gclc)

<400>  29
agaggacaaa ccccaaccac                                                   20


<210>  30
<211>  20
<212>  DNA
<213>  reverse primer (Gclc)

<400>  30
tcgtgcaaag agcctgatgt                                                   20


<210>  31
<211>  20
<212>  DNA
<213>  Artificial sequence

<220>

<223>  forward primer (Gss)

<400>  31
agcgtgccat agagaacgag                                                    20


<210>  32
<211>  20
<212>  DNA
<213>  Artificial sequence

<220>
<223>  reverse primer (Gss)

<400>  32
gcttcccagt tctgtgcgtt                                                    20


<210>  33
<211>  24
<212>  DNA
<213>  Artificial sequence

<220>
<223>  forward primer (Gstm1)

<400>  33
gtcatgccca catagtcttc attc                                               24


<210>  34
<211>  22
<212>  DNA
<213>  Artificial sequence

<220>
<223>  reverse primer (Gstm1)

<400>  34
agttcagggc agacctcaaa tc                                                 22


<210>  35
<211>  21
<212>  DNA
<213>  Artificial sequence

<220>
<223>  forward primer (Gstm6)

<400>  35
ctgagcgttg ctatctcgga g                                                  21


<210>  36
<211>  22
<212>  DNA
<213>  Artificial sequence

<220>
<223>  reverse primer (Gstm6)

<400>  36

ttctgtgtat tccaggagca gc                                                22

<210>  37
<211>  20
<212>  DNA
<213>  Artificial sequence

<220>
<223>  forward primer (Anpep)

<400>  37
cccatcagtg gtttggcaac                                                    20

<210>  38
<211>  23
<212>  DNA
<213>  Artificial sequence

<220>
<223>  reverse primer (Anpep)

<400>  38
catagtcagc acccagaaat tcc                                                23

## Description

### Field of the Invention

The present invention concerns the field of Schizophrenia treatment. More in particular, the present invention is directed to pharmacological methods of treating cognitive deficits in subjects suffering from schizophrenia or at risk of suffering from schizophrenia, as well as to the pharmaceutical products that can be used in these methods.

### Background of the Invention

Schizophrenia (SZ) is a severe psychiatric disorder, affecting approximately 1% of the world's population. Generally, it begins in adolescence or the young adult stage, and lasts the patient's lifetime. It is more frequent and more severe, and has an earlier onset in men than in women. The clinical aspects of SZ are complex, but it is generally accepted that they consist of symptoms from the following three categories: positive symptoms, such as hallucinations and delusions, negative symptoms, such as loss of typical affective functions, and cognitive symptoms, such as impairments in memory and executive functioning. SZ is a heavy personal and socioeconomic burden.

The etiology of SZ is very complex and remains elusive. SZ is caused by a combination of genetic, epigenetic and environmental risk factors. However, the heritability of SZ is 81% as determined by a meta-analysis of twin studies and relatively high compared to the heritability of other complex diseases. The largest genome-wide association study to date showed that 7% of the variability in the risk for SZ in the general population can be attributed to variation in single-nucleotide polymorphisms. Some SZ cases carry a mutation in a specific gene, for example a genetic deletion in the chromosomal region 22q11.2 causes SZ in 25% of its carriers. Whole-exome sequencing has revealed that de novo mutations in PTPRG, TGM5, SLC39A13, BTK and CDKN3 underlie specific SZ cases. However, in most SZ patients an interaction of several genetic variations with environmental risk factors is hypothesized to underlie the etiology of the disorder. A number of environmental risk factors convey SZ risk and include complications around birth, like low birth weight, and use of forceps, preeclampsia and bleeding during pregnancy. In addition, maternal immune activation and maternal stress during pregnancy are well-documented risk factors for SZ. Moreover, paternal age of 30-35 years and older at conception conveys risk for SZ, while the association between maternal age and risk for SZ remains unclear. Likewise, childhood adversity, childhood trauma and adult life events bear SZ risk. Also migration and especially being a refugee increases risk for SZ, although the latter study did not account for trauma or adult-life events as confounding factors. Furthermore, urbanicity increases risk for developing SZ, while exposure to green nature space during childhood decreases SZ risk. Another widely studied risk factor for SZ is cannabis use, and both the amount of cannabis used and the intake of high-potency cannabis are linked to an earlier psychosis onset. Isolated genetic or environmental factors are not sufficient to induce SZ, therefore one must take into account both the accumulative effects of multiple environmental risk factors or multiple genetic risk factors and the interactions between genetic and environmental factors in the etiology of SZ. This is a largely unexplored topic, but a recent study found evidence for an additive effect of genetic risk for SZ and regular cannabis use, sexual abuse, emotional abuse, emotional neglect and bullying. Interestingly, the presence of more than one environmental risk factor causes an earlier onset of SZ, providing evidence for a cumulative effect of environmental risk factors.

Altogether, examining SZ risk factors highlights the complexity of SZ etiology and the necessity for research into the neurobiological causes of this disorder that may eventually allow the development of effective treatment strategies.

Currently, pharmacological therapy focuses on positive symptoms. Both first- and second-generation antipsychotics target the dopamine system and effectively reduce positive symptoms in a subset of patients. Second-generation antipsychotics like haloperidol, chlorpromazine and droperidol are the most commonly prescribed antipsychotic medication despite of their metabolic side effects. If second-generation antipsychotic medication is unsuccessful in reducing psychotic symptoms, first-generation antipsychotics and electroconvulsive therapy can be prescribed.

No treatments targeting negative or cognitive symptoms specifically are currently available in medical practice.

Cognitive deficits in SZ encompass decreases in attention, verbal fluency, processing speed, memory and executive functioning. Executive functions encompass a wide range of cognitive processes that ultimately result in purposeful, goal-directed behavior. Studies using formal neuropsychological instruments have found that many schizophrenia patients have difficulties with most or all of these component processes. For example, patients have a difficult time forming a conceptual framework to understand ambiguous stimuli. If a concept is understood, schizophrenia patients have trouble adapting to changes in the environment that require different behavioral responses. This tendency toward inflexible thinking is found in a number of studies and is highly correlated with occupational difficulties. Another component of executive functioning often found to be impaired in schizophrenia is planning. Perhaps because they encompass so many sub-component processes, the executive functioning tasks are consistently among the best predictors of functional performance. Self-care, social, interpersonal, community, and occupational functions are all associated with executive functioning in schizophrenia. Importantly, executive functions are also associated with treatment success. Impairments in this domain are associated with less engagement in therapy, medication compliance, and longer hospital stays.

The importance of understanding and treating cognitive deficits, in particular executive functioning, in schizophrenia is underscored by the relative lack of treatment success in most aspects of functional status, despite successful treatment of positive symptoms. Most patients nowadays live outside of institutional settings, but their residential and personal status is usually not truly independent. They often rely on financial assistance and clinical support for a range of areas, from work to basic living skills. Even when patients are living in the community, they are still unlikely to succeed in interpersonal relationships, maintain full-time employment, have a stable relationship, or have children.

Therefore, the importance of elucidating the neurobiological mechanisms and developing pharmacological therapies effective in remedying cognitive deficits in SZ cannot be overestimated. Pharmacological enhancement of cognition indeed has been a main field of research already for several decades, investigating different neurotransmitter systems and seemingly reporting as many positive as negative findings. Several meta-analyses and reviews have been conducted regarding potential cognitive enhancers. One of these was conducted by Sinkeviciute et al. (Efficacy of different types of cognitive enhancers for patients with schizophrenia: a meta-analysis. npj Schizophrenia (2018) 4: 22) where the authors reviewed the efficacy of cognitive enhancers acting on the glutamatergic system, the cholinergic system, the serotonergic system, the dopaminergic system, the GABA-ergic system or the noradrenergic system, as well as some agent acting on miscellaneous systems, in improving overall cognitive function and/or specific aspects thereof. For most of the cognitive enhancers (and/or target systems), Sinkeviciute et al. conclude that there are no or only small beneficial effects, mostly with questionable clinical importance.

All in all, to date, there is no clear picture whether any pharmacological treatment can improve cognitive functioning in schizophrenia or specific aspects thereof, such as cognitive flexibility, directly or indirectly by increasing the efficacy of other treatment modalities.

It is the objective of the present invention to, generally stated, provide methods of pharmacological treatments that are effective in improving one or more cognitive functions in schizophrenia patients or specific subsets of schizophrenia patients.

### Summary of the Invention

The present invention is predicated on new experimental findings that have further elucidated the neurobiological underpinnings of prefrontal cortex (PFC) dysfunction in SZ, utilizing the apomorphine-susceptible (APO-SUS) rat model with SZ-relevant features and its phenotypic counterpart the apomorphine-unsusceptible (APO-UNSUS) rat. APO-SUS rats show SZ-relevant behavioural traits and neurobiological characteristics. In contrast to other SZ animal models, APO-SUS rats do not require genetic or pharmacological manipulation to display SZ-relevant behavioural and neurobiological characteristics.

In short, the present inventors have found that medial (m) PFC-dependent cognitive behaviour, including attentional set-shifting, spatial working memory and social behaviour, of APO-SUS rats is impaired as compared to that of APO-UNSUS rats, while cognitive behaviour that is dependent on other brain regions is unaffected. During adolescence, expression levels of myelin-related mRNAs increase in APO-UNSUS mPFC, while such an increase is less pronounced in APO-SUS mPFC, resulting in reduced myelin-related mRNA and protein expression in the mPFC of adult APO-SUS rats. Ultrastructural analyses of the APO-SUS mPFC confirmed a reduced number of myelinated axons in adulthood, whereas no signs of demyelination were observed. Moreover, the unmyelinated axons in the APO-SUS mPFC were found to belong to parvalbumin interneurons, while the number of parvalbumin interneurons was unaltered. Therefore, parvalbumin interneurons were present, but the number that got myelinated was lower in APO-SUS than that in APO-UNSUS mPFC. Interneuron myelination had never before been assessed in an animal model for SZ. The inventor's results support a model in which developmental aberrations during adolescence lead to interneuron hypomyelination in SZ PFC. The inventors further found that oxidative stress plays a significant role in the APO-SUS mPFC, illustrated by a reduced glutathione metabolism and increased numbers of mitochondria. Reduced glutathione metabolism in APO-SUS mPFC was observed throughout post-natal life and as such preceded hypomyelination that occurred merely during adolescence.

In their experiments, the inventors discovered that treatment of APO-SUS rats during postnatal development with the antioxidant N-acetyl cysteine restored blood plasma levels of glutathione, mRNA expression of glutathione-related genes and mitochondria numbers in APO-SUS mPFC. The antioxidative effects of N-acetyl cysteine were found to rescue interneuron hypomyelination. Moreover, N-acetyl cysteine treatment of APO-SUS rats resulted in a significant improvement in cognitive inflexibility in the extra-dimensional set-shifting task. This task is equivalent to the Wisconsin card sorting test that is used to reveal cognitive inflexibility in SZ patients.

All in all, the findings underlying the present application indicate that N-acetyl cysteine treatment will be efficacious in preventing cognitive deficits in individuals at high-risk to develop SZ. The acquired neurobiological insights support N-acetyl cysteine treatment, especially chronic N-acetyl cysteine treatment, optionally in combination with behavioral therapy (such as sociability training, physical exercise and environmental enrichment), as a preventative strategy and an appealing remedy to alleviate PFC hypomyelination and cognitive symptoms in SZ.

To the best of the inventor's knowledge no prior art document has ever presented data confirming the efficacy of N-acetyl cysteine in improving cognitive symptoms in general and/or cognitive flexibility in particular in SZ patients. Breier et al. (Effects of 12-month, double-blind N-acetyl cysteine on symptoms, cognition and brain morphology in early phase schizophrenia spectrum disorders. Schizophr Res. 2018 Sep; 199:395-402) assessed the effects of N-acetyl cysteine (3600mg/day) in a 52-week, double-blind, placebo controlled trial on symptoms and cognition in early phase schizophrenia spectrum disorders. Symptoms were assessed by the Positive and Negative Syndrome Scale (PANSS) and Brief Assessment of Cognition in Schizophrenia (BACS), which assesses certain domains of cognition, including verbal memory, working memory, processing speed, and reasoning/problem solving (based on the following tests: Verbal Memory (VM), Digit Sequencing (DS), Token Motor Task (TMT), Semantic Fluency (SF), Symbol Coding (SC), and Tower of London (TOL)). Breier et al. conclude that in individuals with early phase schizophrenia spectrum disorders, 52 weeks of N-acetyl cysteine treatment improved PANSS total and negative symptoms but failed to improve PANSS positive symptom scores, as well as PANSS disorganized thought symptoms, while the treatment failed to improve cognitive functioning, including working memory and processing speed. The study by Breier et al. did not include any assessment of PFC-dependent cognitive functioning.

The present inventors are the first to provide evidence, based on PFC-directed cognitive tests, that N-acetyl cysteine treatment improves cognitive outcome in an animal model and affects cellular and physiological mechanisms implicated in cognitive dysfunction of SZ patients, notably it normalizes the expression levels of myelin-related mRNAs and myelination in mPFC. These finding imply that N-acetyl cysteine will have utility in pharmacological methods of improving cognitive symptoms, in particular cognitive flexibility, in SZ patients. Thus, the invention, stated very generally, concerns methods of treating cognitive symptoms in subjects in need of such treatment, said method involving the administration of a composition comprising N-acetyl cysteine or a pharmaceutically acceptable derivative thereof. More in particular, the invention concerns the following aspects.

An aspect of the invention concerns a method of treating and/or preventing one or more cognitive symptoms, in particular one or more PFC-dependent cognitive symptoms, such as cognitive inflexibility, in a subject suffering from schizophrenia or at risk of suffering from schizophrenia, said method comprising the administration of a pharmaceutical composition comprising N-acetyl cysteine or a pharmaceutically acceptable derivative thereof as defined herein, preferably N-acetyl cysteine or a salt thereof.

An aspect of the invention concerns a pharmaceutical composition comprising N-acetyl cysteine or a pharmaceutically acceptable derivative thereof as defined herein, preferably N-acetyl cysteine or a salt thereof, for use in a method of treating and/or preventing one or more cognitive symptoms, in particular one or more PFC-dependent cognitive symptoms, such as cognitive inflexibility, in a subject suffering from schizophrenia or at risk of suffering from schizophrenia.

An aspect of the invention concerns the use of a composition comprising N-acetyl cysteine or a pharmaceutically acceptable derivative thereof, preferably N-acetyl cysteine or a salt thereof, in the manufacture of a medicament for use in a method of treating and/or preventing one or more cognitive symptoms, in particular one or more PFC-dependent cognitive symptoms, such as cognitive inflexibility in a subject suffering from schizophrenia or at risk of suffering from schizophrenia.

An aspect of the invention concerns a pharmaceutical composition, preferably in unit dose form, said composition comprising N-acetyl cysteine or a pharmaceutically acceptable derivative thereof as defined herein, preferably N-acetyl cysteine or a salt thereof, in the manufacture of a medicament for use in a method of treating and/or preventing one or more cognitive symptoms, in particular one or more PFC-dependent cognitive symptoms, such as cognitive inflexibility in a subject suffering from schizophrenia or at risk of suffering from schizophrenia, wherein said pharmaceutical composition is suitable and/or optimized for use in the present methods of treatment.

An aspect concerns a kit comprising a package containing a plurality of the unit dosage forms of the invention as well as a leaflet containing printed instructions to repeatedly self-administer said unit dosage forms in order to treat and/or prevent one or more cognitive symptoms, in particular one or more PFC-dependent cognitive symptoms, such as cognitive inflexibility, in a subject suffering from schizophrenia or at risk of suffering from schizophrenia.

It will be understood that these aspects of the invention all involve the same compositions, the same methods of treatment, the same subjects, etc. unless specifically stated otherwise. Specific details and preferred embodiments of the afore-mentioned methods as well as of the compositions and pharmaceutical kits used therein will become evident to those skilled in the art on the basis of the following detailed description and the appended experimental part.

### Detailed description of the Invention

### Pharmaceutical Composition

Compositions that can be used in accordance to the present invention comprise an N-acetyl cysteine or a pharmaceutically acceptable N-acetyl cysteine derivative.

N-acetyl cysteine (NAC) (also referred to as N-acetylcysteine) as used herein refers to the L-isomer of N-acetyl cysteine, which is the N-acetylated derivative of the naturally occurring amino acid L-cysteine. The molecular formula of the compound is C₅H₉NO₃S, having the following structural formula (I):

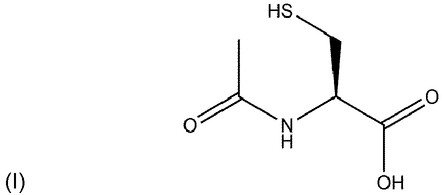

As used herein, the term "derivative" refers to a compound having a structure derived from the structure of a parent compound, e.g. N-acetyl cysteine, which is sufficiently similar to that of the parent compound such that based upon that structural similarity, the derivative would be expected by one skilled in the art to exhibit the same or similar pharmacological activity and to have the same therapeutic utility as the parent compound, or it would be expected by one skilled in the art to generate the therapeutic compound in vivo through dissolution, dissociation or metabolisation, thereby achieving the same or similar pharmacological activity and therapeutic utility as the parent compound. Exemplary derivatives include salts, esters, thioesters, amides and salts of esters, thioesters or amides.

In a preferred embodiment of the present invention, compositions are provided comprising an N-acetyl cysteine (derivative) selected from the group of compounds represented by formula (II), pharmaceutically acceptable salts thereof, compounds represented by formula (III) and pharmaceutically acceptable salts thereof: 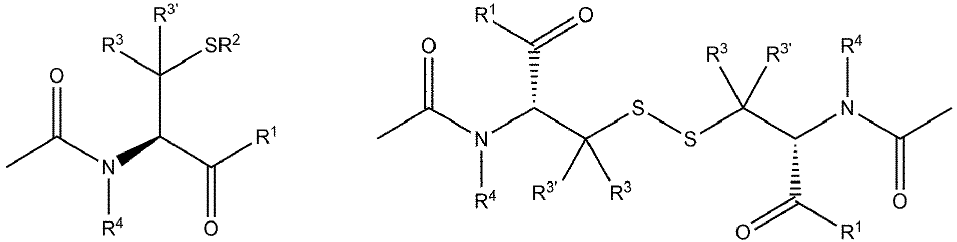 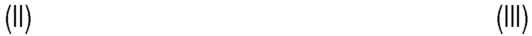 wherein:
-R¹ represents -OH, -NH₂, -NHR⁵ or -OR⁵ wherein -R⁵ represents a straight-chain or branched C₁₋₆-alkyl, a straight-chain or branched C₂₋₆-alkenyl, phenyl or phthalidyl, preferably -R¹ represents -OH, - NH₂ -OCH₃, -OCH₂CH₃, -OCH₂CH₂CH, -OCH(CH₃)CH₃ or -CH₂CHCH₂, more preferably -R¹ represents -OH, -NH₂ -OCH₃ or -OCH₂CH₃, still more preferably -R¹ represents -OH or -NH₂;
-R² represents -H, -R⁶ or -COR⁶, wherein -R⁶ represents a straight-chain or branched C₁₋₆-alkyl, a straight-chain or branched C₂₋₆-alkenyl or phenyl, preferably -R² represents -H, -CH₃, -CH₂CH₃, - COCH₃, -COCH₂CH₃, or -CH₂CHCH₂, more preferably -R² represents -H, -COCH₃ or -CH₃, still more preferably -R² represents -H;
-R³ and -R³' individually represent -H or -CH₃, preferably -R³ and -R³' are the same, more preferably -R³ and -R³' represent -H; and
-R⁴ represents -H, -R⁷ or -COR⁷, wherein -R⁷ represents a straight-chain or branched C₁₋₆-alkyl, a straight-chain or branched C₂₋₆-alkenyl or phenyl, preferably -R⁴ represents -H, -CH₃, -CH₂CH₃, - COCH₃, -COCH₂CH₃ or -CH₂CHCH₂, more preferably -R⁴ represents -H, -COCH₃ or -CH₃, still more preferably -R⁴ represents -H.

In a first particularly preferred embodiment of the present invention, compositions are provided comprising N-acetyl cysteine (NAC) or a pharmaceutically acceptable salt thereof.

In a second particularly preferred embodiment of the present invention, compositions are provided comprising N-acetyl cysteine amide (NACA) or a pharmaceutically acceptable salt thereof. N-acetyl cysteine amide is more hydrophobic and lipophilic than N-acetyl cysteine, allowing higher ability to cross the blood brain barrier, which can further enhance treatment efficacy.

The term "pharmaceutically acceptable salt" as used herein, refers to any salt, e.g., obtained by reaction with an acid or a base, of a Compound of the Disclosure that is physiologically tolerated in the human body. Examples of pharmaceutically acceptable salts include inorganic and organic acid addition salts and basic salts. The pharmaceutically acceptable salts include, but are not limited to, metal salts such as sodium salt, potassium salt, cesium salt and the like; alkaline earth metals such as calcium salt, magnesium salt and the like; organic amine salts such as triethylamine salt, ethanolamine salt, triethanolamine salt, dicyclohexylamine salt, and the like; inorganic acid salts such as hydrochloride, hydrobromide, phosphate, sulphate and the like; organic acid salts such as citrate, lactate, tartrate, maleate, fumarate, mandelate, acetate, dichloroacetate, trifluoroacetate, oxalate, formate and the like; and amino acid salts such as arginate, asparginate, glutamate and the like.

The compositions of the invention may comprise one or more additional ingredients. In a preferred embodiment, the composition comprises one or more additional ingredients that render the composition suitable for administration to subjects in need thereof, such as one or more carriers and/or excipients. As is known by those of average skill in the art, the appropriate choice of excipients is largely dependent on the pharmaceutical form and route of administration preferred. The compositions of the invention can be formulated for a variety of routes of administration, oral administration being particularly preferred. It is within the purview of those of average skill in the art to conceive and develop suitable formulations, relying on the common general knowledge as reflected in text books such as Remington's Pharmaceutical Sciences (Meade Publishing Co., Easton, Pa., 20.sup.th Ed., 2000), the entire disclosure of which is herein incorporated by reference, and routine development efforts.

In accordance with the various aspects of the invention, the composition is preferably provided in a unit dosage form. The term 'unit dosage form' refers to a physically discrete unit suitable as a unitary dosage for human subjects, each unit containing a predetermined quantity of active material calculated to produce the desired therapeutic effect in association with any suitable pharmaceutical carrier(s) and/or excipient(s). Exemplary, non-limiting unit dosage forms include a tablet (e.g., a chewable tablet), caplet, capsule (e.g., a hard capsule or a soft capsule), lozenge, film, strip, gelcap as well as any metered volume of a solution, suspension, syrup or elixir or the like, which may be contained, for instance in a vial, syringe, applicator device, sachet, spray, micropump etc. In accordance with particularly preferred embodiments of the invention, the unit dosage form, is a unit dosage form that is suitable for oral administration.

In accordance with the various aspects of the invention, the composition is provided in a unit dosage form as defined herein, which unit dosage form comprises the N-acetyl cysteine (derivative) in an amount that is equipotent to at least about 250 mg of N-acetyl cysteine, more preferably at least about 500 mg of N-acetyl cysteine, at least about 750 mg of N-acetyl cysteine, at least about 1000 mg of N-acetyl cysteine, at least about 1200 mg of N-acetyl cysteine, at least about 1400 mg of N-acetyl cysteine, or at least about 1500 mg of N-acetyl cysteine. In accordance with the various aspects of the invention, the composition is provided in a unit dosage form comprising the N-acetyl cysteine (derivative) in an amount that is equipotent to about 5000 mg of N-acetyl cysteine or less, more preferably about 4000 mg of N-acetyl cysteine or less, about 3500 mg of N-acetyl cysteine or less, about 3000 mg of N-acetyl cysteine or less, about 2500 mg of N-acetyl cysteine or less, about 2250 mg of N-acetyl cysteine or less, or about 2000 mg of N-acetyl cysteine or less. In accordance with the various aspects of the invention, the composition is provided in a unit dosage form comprising the N-acetyl cysteine (derivative) in an amount that is equipotent to about 500-4000 mg of N-acetyl cysteine, more preferably to about 750-3500 mg of N-acetyl cysteine, to about 1000-3000 mg of N-acetyl cysteine, or to about 1200-2500 mg of N-acetyl cysteine, e.g. to about 1800 mg of N-acetyl cysteine.

As used herein, the term "equipotent" means equally potent or equally capable of producing a pharmacologic effect of certain intensity. It is common in the art, to express an amount of a given pharmaceutically active compound as the quantity required to produce an effect quantitatively comparable to the effect obtained with a defined amount of a reference compound, which typically belongs to the same class of pharmaceutically active compounds. It is also common in the art to refer to amounts of a given compound 'equivalent' to a specified amount of a reference compound. For example, if the composition comprises a salt of N-acetyl cysteine the amount of said salt to be administered and/or to be incorporated into a unit dose form needs to be adjusted to take account of the molecular weight difference between the free base and salt form. This is, for instance, customary practice in expressing dose amounts in the label and/or product information of authorized medicinal products comprising a salt form of an active compound that is also used in free base form. In this context, the term 'equipotent' is deemed synonymous to the term 'equivalent'.

In accordance with the various aspects of the invention, the composition is provided in a unit dosage form comprising N-acetyl cysteine in an amount of at least about 250 mg, more preferably at least about 500 mg, at least about 750 mg, at least about 1000 mg, at least about 1250 mg, or at least about 1500 mg. In accordance with the various aspects of the invention, the composition is provided in a unit dosage form comprising N-acetyl cysteine in an amount of about 5000 mg or less, more preferably about 4000 mg or less, about 3500 mg or less, about 3000 mg or less, about 2500 mg or less, about 2250 mg or less, or about 2000 mg or less. In accordance with the various aspects of the invention, the composition is provided in a unit dosage form comprising N-acetyl cysteine in an amount within the range of about 500-4000 mg, more preferably about 750-3500 mg, about 1000-3000 mg, about 1200-2500 mg, e.g. about 1800 mg.

In accordance with certain embodiments of the invention, the composition comprises a second therapeutic ingredient that has efficacy in the treatment of one or more symptoms associated with schizophrenia. In one embodiment such a second therapeutic ingredient is selected from the group of psychotropic agents, such as an anti-psychotic agent, e.g. an anti-psychotic agent selected from the group consisting of benperidol. bromperidol, droperidol, haloperidol, moperone, pipamperone, timiperone, fluspirilene, penfluridol, pimozide, phenothiazines, acepromazine, chlorpromazine, cyamemazine, dixyrazine,fluphenazine, levomepromazine, mesoridazine, perazine, pericyazine, perphenazine, pipotiazine, prochlorperazine, promazine, promethazine, prothipendyl, thioproperazine, thioridazine, trifluoperazine, triflupromazine, thioxanthenes, chlorprothixene, clopenthixol, flupentixol, thiothixene, zuclopenthixol, clotiapine, loxapine,prothipendyl, carpipramine, clocapramine, molindone, mosapramine, sulpiride, sultopride, veralipride, amisulpride, amoxapine, aripiprazole, asenapine, cariprazine, clozapine, blonanserin, iloperidone, lurasidone, melperone, nemonapride, olanzapine, paliperidone, perospirone, quetiapine, remoxipride, risperidone, sertindole, sultopride, trimipramine, ziprasidone, zotepine, brexpiprazole, ITI-007, pimavanserin and RP5063.

In other embodiments, said second therapeutic agent is selected from the group of agents acting on the glutamatergic system, agents acting on the cholinergic system, agents acting on the serotonergic system, agents acting on the dopaminergic system, agents acting on the GABA-ergic system, agents acting on the adrenergic system and agents acting on the noradrenergic system. Particular examples are D-serine, D-cycloserine, Ampakine CX516, amantadine, glycine, d-cyclosrine, lamotigrine, memantine, pregnenlone, minocycline, L-carnosine, benzoate, mirtazapine, duloxetine, dimebon, latrep, donepezil, nicotine, rivastigmine, galantamine, DMXB-A, tropisetron, varenicline, AZD3480, CDP-choline, TC-5619, RG3487, enenicline, TC-5619, ABT-126, tandospirone, mianserin, citalopram, buspirone, ondansetron, duloxetine, dimebon, latrepirdine, fluvoxamine, AVN-211, d-amphetamine, entacapone, DAR-0100A, MK-0777, guanfacine, atomoxetine, reboxetine, modafinil, sildenafil, armodafil, davunetide, rosiglitazone, modafinil and raloxifene.

In other embodiments, said second therapeutic agent is selected from the group of agents that increase plasma levels of glutathione, such as curcumin, selenium, silymarin, vitamin C and vitamin E.

In other embodiments, said second therapeutic agent is selected from the group of agents that exert antioxidative effects, such as vitamins, quercetin, selenium, amino acids, nucleotides, antimicrobial peptides, bacteria, polyunsaturated fatty acids, oligosaccharides, and plant extracts having antioxidative effects.

In other embodiments, said second therapeutic agent is selected from the group of agents that has efficacy in the treatment of the positive symptoms associated with schizophrenia, such as clozapine, chlorpromazine, haloperidol, perphenazine, risperidone, quetiapine, ziprasidone, aripiprazole, paliperidone, asenapine, iloperidone, lurasidone.

### Therapeutic indications

As explained herein before, the methods of the invention are intended and/or suited for the treatment and/or prevention of one or more cognitive symptoms in a subject in need thereof, in particular PFC-dependent cognitive symptoms, such as cognitive flexibility.

The terms "treat", "treating" or "treatment" as used herein, when appearing in conjunction with a specific disorder or symptom (for example: "method of treating disease ...") refers to a method of alleviating or abrogating said disorder and/or accompanying symptoms. The terms "prevent", "preventing" or "prevention", as used herein, refer to a method of barring a subject from acquiring a disorder and/or its attendant symptoms. In certain embodiments, the terms "prevent", "preventing" or "prevention" refer to a method of reducing the risk of acquiring a disorder and/or accompanying symptoms. The terms "treat", "treating" or "treatment", when used in relation to a patient or subject (for example: "method of treating a subject"), typically refers to the act of administering a therapeutic compound to said patient or subject for whatever therapeutic and/or prophylactic purpose.

The term 'cognitive symptoms' is well understood by those skilled in the field. It refers to (deficits in) the abilities of a subject in one of the following cognitive domains (according to DSM-5): perceptual-motor function (including visual perception, visuoconstructional reasoning and perceptual moto-coordination), language (including object naming, word finding, fluency, grammar and syntax and receptive language), learning and memory (including free recall, cued recall, recognition memory, semantic and autobiographical, long-term memory and implicit learning), social cognition (recognition of emotions, theory of mind, insight), complex attention (sustained attention, divided attention, selective attention, processing speed) and executive functioning (planning, decision-making, working memory, responding to feedback, inhibition and flexibility). In one particularly preferred embodiment, the method of the invention is intended and/or suited for treating executive dysfunction or deficits and complex attention deficits, for improving executive functioning, for maintaining executive functioning and/or for preventing executive dysfunction or deficits, in a subject suffering from schizophrenia. The term 'executive functioning' refers to a set of cognitive abilities that control and regulate other abilities and behaviors. Executive functions are high-level abilities that influence more basic abilities like attention, memory and motor skills. The executive functions are necessary for goal-directed behavior, and include the ability to initiate and stop actions, to monitor and change behavior as needed, and to plan future behavior when faced with novel tasks and situations. Executive functions allow one to anticipate outcomes and adapt to changing situations. The ability to form concepts and the ability to think abstractly are often considered components of executive function. In an embodiment of the invention, the method is intended and/or suited for the treatment and/or prevention of dysexecutive syndrome.

In one particularly preferred embodiment, the method of the invention is directed at the treatment of cognitive inflexibility, at improving cognitive flexibility, at preventing cognitive inflexibility and/or at maintaining cognitive flexibility, in a subject suffering from schizophrenia. In the field, several tests are known that are generally considered suitable to assess cognitive flexibility, including the Wisconsin Card-Sorting Test (WCST), the Trail Making Test (TMT) Part B and verbal fluency tests. In a preferred embodiment of the invention, the method is directed at the treatment of cognitive inflexibility and/or at improving cognitive flexibility, as reflected by a decrease in the total number of errors (total number of errors and/or perseverative errors) in the WCST during the treatment period, e.g. a decrease in the total number of errors (total number of errors and/or perseverative errors) of at least 5%; and/or the prevention of cognitive inflexibility and/or at maintaining cognitive flexibility, as reflected by a more or less constant total number of errors (total number of errors and/or perseverative errors) in the WCST during the treatment period, e.g. an increase or decrease in the number of errors (total number of errors and/or perseverative errors) in the WCST of less than 5%.

In a particularly preferred embodiment of the present invention, the method is directed at the treatment and/or prevention of one or more PFC-dependent cognitive symptoms. The term 'PFC-dependent cognitive symptoms' as used herein typically refers to impairments in cognitive functions dependent on (alterations in) the prefrontal cortex ('PFC'). In preferred embodiments of the present invention, the one or more PFC-dependent cognitive symptoms are selected from a deficit in cognitive flexibility, social cognition, processing speed, response to novelty or behavioral-emotional self-regulation, most preferably a cognitive flexibility deficit. A deficit in cognitive flexibility (i.e., the inability to adapt to changes in the ruleset of a task) includes a deficit in attentional shifting, such as intra-dimensional set-shifting, extra-dimensional set-shifting and (probabilistic) reversal learning. Methods of measuring cognitive flexibility include the A-not-B test, Dimensional Change Card Sorting Test, Multiple Classification Card Sorting Test, Wisconsin Card Sorting Test, and the Stroop Test.

### Subjects

As explained herein before, the methods of the invention are directed at the treatment and/or prevention of the aforementioned symptoms and conditions in subjects suffering from schizophrenia or at risk of suffering from schizophrenia.

The DSM-5 outlines the criteria to make a diagnosis of schizophrenia. In preferred embodiments of the invention, the subject is diagnosed as suffering from schizophrenia, in accordance with said DMS-5 criteria. In a preferred embodiment of the invention, the subject experiences cognitive symptoms of schizophrenia, especially executive dysfunction or deficits, more particularly a deficit in cognitive flexibility. In a preferred embodiment of the invention, the subject has low white-matter integrity. In a preferred embodiment of the invention, the subject suffers from a deficit in PFC myelination or from PFC hypomyelination. In a preferred embodiment of the invention, the subject has low blood plasma levels of glutathione. In a preferred embodiment the subject has low mRNA expression of glutathione-related genes. In preferred embodiments of the invention, the subject is genetically predisposed to schizophrenia, e.g. as a consequence of *de novo* mutations in PTPRG, TGM5, SLC39A13, BTK or CDKN3. In preferred embodiments of the invention, the subject is behaviorally and/or environmentally predisposed to schizophrenia, e.g. as a consequence of complications around the subject's birth, like low birth weight and use of forceps, preeclampsia and bleeding during pregnancy; maternal immune activation and maternal stress during pregnancy; paternal age of 30-35 years and older at conception, childhood adversity, childhood trauma, adult life events or cannabis use. In a preferred embodiment of the invention, the subject to be treated is a subject experiencing symptoms of schizophrenia.

In accordance with the invention the subject is typically a human. In one embodiment of the invention, the subject is human male. In another embodiment of the invention, the subject is human female. Schizophrenia can in principle occur and/or manifest at any stage in life, with the earliest onset age typically being around 12. Hence, the subject can typically have any age above 12 years, such an age ranging from 12-99 years. In particularly preferred embodiments of the invention, the treatment is started during the early stages of schizophrenia development. Hence, preferably, the subject has an age of 12 years or older, e.g. 13 years or older, 14 years or older, 15 years or older, 16 years or older, 17 years or older, 18 years or older, 19 years or older or 20 years or older. In preferred embodiments of the invention the subject has an age of 50 years or younger, e.g. 45 years or younger, 40 years or younger, 35 years or younger, 30 years or younger, 29 years or younger, 28 years or younger, 27 years or younger, 26 years or younger or 25 years or younger.

### Treatments

The various aspects of the present invention as defined herein, all relate to treatments involving the administration, typically the repeated administration, of a composition comprising the N-acetyl cysteine (derivative), preferably any composition as defined herein before.

In particularly preferred embodiments of the invention, the treatment comprises the administration of the composition through the enteral route, such as through the oral route.

In particularly preferred embodiments of the invention, the treatment comprises the administration of the N-acetyl cysteine (derivative) containing composition at a daily dose that is equipotent to at least about 500 mg of N-acetyl cysteine, more preferably at least about 1000 mg of N-acetyl cysteine, at least about 1500 mg of N-acetyl cysteine, at least about 2000 mg of N-acetyl cysteine, at least about 2400 mg of N-acetyl cysteine, at least about 2800 mg of N-acetyl cysteine, or at least about 3000 mg of N-acetyl cysteine. In particularly preferred embodiments of the invention, the treatment comprises the administration of the composition at a daily dose that is equipotent to about 10,000 mg of N-acetyl cysteine or less, more preferably about 8000 mg of N-acetyl cysteine or less, about 7000 mg of N-acetyl cysteine or less, about 6000 mg of N-acetyl cysteine or less, about 5000 mg of N-acetyl cysteine or less, about 4500 mg of N-acetyl cysteine or less, or about 4000 mg of N-acetyl cysteine or less. In particularly preferred embodiments of the invention, the treatment comprises the administration of the composition at a daily dose that is equipotent to about 1000-8000 mg of N-acetyl cysteine, more preferably to about 1500-7000 mg of N-acetyl cysteine, to about 2000-6000 mg of N-acetyl cysteine, to about 2400-5000 mg of N-acetyl cysteine, e.g. to about 3600 mg of N-acetyl cysteine. As will be understood, particularly preferred treatment comprises the administration of N-acetyl cysteine at a daily dose of at least about 500 mg, more preferably at least about 1000 mg, at least about 1500 mg, at least about 2000 mg, at least about 2400 mg, at least about 2800 mg or at least about 3000 mg and/or at a daily dose of about 10,000 mg or less, more preferably about 8000 mg, about 7000 mg or less, about 6000 mg or less, about 5000 mg or less, about 4500 mg or less, or about 4000 mg or less. In particularly preferred embodiments of the invention, the treatment comprises the administration of N-acetyl cysteine at a daily dose of about 1000-8000 mg, more preferably about 1500-7000 mg of N-acetyl cysteine, about 2000-6000 mg or about 2400-5000 mg, e.g. about 3600 mg of N-acetyl cysteine.

In accordance with the invention, the daily dose indicated herein may be contained in a single unit dosage form as well as in a plurality of unit dosage forms. For example, in one particularly preferred embodiment, the method comprises the administration of 2 unit dose forms, each comprising approximately half of the daily dose as indicated above, at certain pre-determined moments during the day. Embodiments wherein unit dosage forms are used comprising higher amounts of the N-acetyl cysteine (derivative) than the daily dose indicated herein are also contemplated. This may e.g. involve the use of extended release dosage forms that remain in the body and keep releasing the active ingredient for a sufficiently long time.

In particularly preferred embodiments of the invention, the treatment comprises the repeated administration of the N-acetyl cysteine (derivative) containing composition, preferably in the daily dose as defined herein before. In particularly preferred embodiments of the invention, the treatment comprises the repeated administration of the N-acetyl cysteine (derivative) containing composition, preferably in the daily dose as defined herein before, at a frequency of at least once weekly, at least once every three days, at least once every two days, at least once every day. It will be understood by those skilled in the art, based on the teachings presented herein, that repeated administration preferably involves the administration of the N-acetyl cysteine (derivative) containing composition at fixed time points during the day and/or at regular intervals, such as every morning, every afternoon or every evening in case of once daily administration; or every morning and every evening in case of twice daily administration; or every morning, every afternoon and every evening in case of administration three times daily; or every other morning, every other afternoon or every other evening, in case of administration once every two days.

In particularly preferred embodiments of the invention, the treatment comprises the repeated administration of the N-acetyl cysteine (derivative) containing composition, preferably in accordance with the above-defined regimens, during a period of at least one month, at least three months, at least four months, at least six months, at least nine months, at least one year, at least two year or at least three year. There is no particular upper limit; treatment may be continued for as long as it is deemed beneficial to the subject's overall health and well-being (as determined by appropriately qualified healthcare professional).

The findings of the present inventors support start of the treatment as soon as possible for maximum efficacy. Hence, in accordance with preferred embodiments of the invention, the treatment of the subject is started as soon as the subject is first diagnosed with schizophrenia, e.g. within one day from said moment, within two days from said moment, within three days from said moment, within four days from said moment, within five days from said moment, within six days from said moment or within seven days from said moment. In accordance with particularly preferred embodiments of the present invention, treatment starts shortly after the moment the subject experiences the first symptoms of schizophrenia, e.g. within one day from said moment, within two days from said moment, within three days from said moment, within four days from said moment, within five days from said moment, within six days from said moment or within seven days from said moment. In other preferred embodiments of the invention, the treatment starts when the subject has an age of 12 years or older, e.g. 13 years or older, 14 years or older, 15 years or older, 16 years or older, 17 years or older, 18 years or older, 19 years or older or 20 years or older. In preferred embodiments of the invention, the treatment starts when the subject has an age of 50 years or younger, e.g. 45 years or younger, 40 years or younger, 35 years or younger, 30 years or younger, 29 years or younger, 28 years or younger, 27 years or younger, 26 years or younger or 25 years or younger. In accordance with certain embodiments of the invention, the treatment is started irrespective of the occurrence of noticeable schizophrenia symptoms, e.g. when it is established based on certain genetic, behavioral and/or environmental factors that the subject is predisposed to schizophrenia.

In some embodiments, the N-acetyl cysteine (derivative) treatment according to the invention may be used in conjunction with other treatments that may aid in the prevention or treatment of schizophrenia symptoms and/or improve the overall health and well-being of subjects suffering from schizophrenia. Hence, in embodiments of the invention, methods of treatment as defined herein are provided, said method further comprising the co-treatment with a second (or further) therapeutic agent selected from the group consisting of psychotropic agents, anti-psychotics, agents acting on the glutamatergic system, agents acting on the cholinergic system, agents acting on the serotonergic system, agents acting on the dopaminergic system, agents acting on the GABA-ergic system, agents acting on the adrenergic system and agents acting on the noradrenergic system, agents that increase plasma levels of glutathione and agents that exert antioxidative effects, as described herein elsewhere. In one particularly preferred embodiment of the invention methods are provided wherein the subject is administered an agent that has efficacy in the treatment of the positive symptoms associated with schizophrenia thereof in a therapeutically effective dose, during at least part of the treatment with the N-acetyl cysteine (derivative) as defined herein above.

### Pharmaceutical Kits

A fourth aspect of the invention concerns a pharmaceutical kit comprising a package containing a plurality of unit dosage forms as defined herein before, comprising an effective amount of N-acetyl cysteine (derivative), and a leaflet containing printed instructions to repeatedly self-administer said unit dosage forms by oral ingestion so as to attain one or more of the therapeutic and/or prophylactic objectives defined herein.

In accordance with preferred embodiments of the invention, the pharmaceutical kit comprises a container, such as a cardboard box, holding one or more blister packs, said one or more blister packs containing a plurality of solid unit dosage forms as defined herein before, preferably a plurality of capsules as defined herein before. In particularly preferred embodiments of the invention, the pharmaceutical kit comprises at least 5, at least 8, at least 10, at least 12 of at least 15, at least 20, at least 30 or at least 40 of said unit dosage forms.

In accordance with the invention, the pharmaceutical kit comprises a leaflet inserted into the container, typically a patient information leaflet containing printed information, which information may include a description of the form and composition of the unit dosage forms contained in the kit, an indication of the therapeutic indications for which the product is intended, instructions as to how the product is to be used and information and warnings concerning adverse effects and contraindications associated with the use. It will be understood by those of average skill in the art, based on the information presented herein, that the leaflet will typically contain the information concerning the therapeutic indications, uses, treatment regimens, etc. as described here above in relation to the methods of treatment of the present invention.

As used herein, the term "comprise" and variations of the term, such as "comprising" and "comprises," are not intended to exclude other additives, components, integers or steps. The terms "a," "an," and "the" and similar referents used herein are to be construed to cover both the singular and the plural unless their usage in context indicates otherwise. Furthermore, to the extent that the term "include," "have," or the like is used in the description or the claims, such term is intended to be inclusive in a manner similar to the term "comprise" as "comprise" is interpreted when employed as a transitional word in a claim.

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed; rather, the phrase allows a meaning that includes at least one of any one of the items, and/or at least one of any combination of the items, and/or at least one of each of the items. By way of example, the phrase "at least one of A, B, and C" refers to only A, only B, or only C; any combination of A, B, and C; and/or at least one of each of A, B, and C.

The term "about", whenever used in conjunction with a number or value, generally means within the tolerance range of the equipment used to determine the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51% to about 100%, for example.

Throughout this text, the use of words in brackets as part of a given term usually means that the wording within brackets specifies a possible option or a possible meaning and that each option or meaning is encompassed by the term. By way of example, the term "N-acetyl cysteine (derivative)" should be read as "N-acetyl cysteine or an N-acetylcysteine derivative".

All publications and patents cited in this disclosure are incorporated by reference in their entirety. To the extent the material incorporated by reference contradicts or is inconsistent with this specification, the specification will supersede any such material. The citation of any references herein is not an admission that such references are prior art to the present invention.

The foregoing description is provided to enable a person skilled in the art to practice the various embodiments described herein. While the subject technology has been particularly described with reference to the various embodiments, it should be understood that these are for illustration purposes only and should not be taken as limiting the scope of the subject technology. Those skilled in the art will recognize, or be able to ascertain using no more than routine experimentation, many equivalents to the specific embodiments of the invention described herein.

The various aspects and embodiments as defined in the foregoing will be further illustrated by means of the following non-limiting examples.

### Examples

### Example 1: Neurobiological basis and repair of interneuron hypomyelination and cognitive inflexibility in rat model for schizophrenia

To explore the neurobiological underpinnings of mPFC dysfunction in SZ, a well-characterized, idiopathic rat model with SZ-relevant features was selected, namely the apomorphine-susceptible (APO-SUS) rat along with its phenotypic counterpart, the apomorphine-unsusceptible (APO-UNSUS) rat. APO-SUS rats do not require genetic or pharmacological manipulation to display SZ-relevant behavioural characteristics. These characteristics are related to the positive, negative and cognitive symptom domains, and include reduced prepulse inhibition, increased exploratory behaviour, dopamine-induced stereotypic behaviour, reduced latent inhibition, reduced sucrose preference and memory deficits. Furthermore, the APO-SUS rats display neurobiological features similar to those found in SZ, e.g., a hyperactive hypothalamus-pituitary-adrenal (HPA)-axis and elevated dopamine D2-receptor binding in the nucleus accumbens.

### Methods

### Animal model

Generation of the APO-SUS and APO-UNSUS rat lines has already been described in detail. Briefly, rats were selectively bred from an outbred Nijmegen Wistar rat population that displayed stereotyped behaviour upon injection of apomorphine (APO-SUS rats). The same selective breeding was performed with the rats that showed a weak apomorphine-induced stereotypy (APO-UNSUS rats). Apomorphine injection and behavioural selection were only performed with the first 15 generations of APO-SUS and APO-UNSUS rats. In the subsequent breedings, APO-SUS rats displayed SZ-relevant features without this pharmacological treatment. In this study, naïve male APO-SUS and APO-UNSUS rats from the 32th-44rd generation were used. Rats were housed in pairs in a temperature- and humidity-controlled room with a 12-h light-dark cycle (lights on at 7.00 a.m.) and ad *libitum* access to water and standard laboratory chow (V1534-703, SSNIFF, Germany), unless otherwise indicated. Animal experiments were approved by the Animal Ethics Committee of Radboud University Nijmegen Medical Centre, Nijmegen, the Netherlands, and were conducted in accordance with Dutch legislation (Herziene Wet op Dierproeven, Art 10.a.2, 2014).

### Micropunching

For micropunching, naïve P21 (+/- 1 day), P60 (+/- 1 day), P90 (+/- 1 day), P120 (+/- 1 day) and P365 (+/- 14 days) APO-SUS and APO-UNSUS rats were sacrificed by direct decapitation and brains were isolated, frozen on dry ice and stored at -80 °C. Micropunching was performed in a cryostat (Leica) at -15 °C and the Paxinos and Watson rat brain atlas was taken as a reference to aid the dissection. mPFC was collected with a 1.20 or 2.00-mm punch needle (Harris) from 300 µm coronal sections at Bregma 4.00- 2.20. Dissected tissues were immediately frozen on dry ice and stored at -80 °C until further analysis.

### RNA isolation

RNA for RNA-seq analysis was extracted from P365 APO-SUS and APO-UNSUS mPFC with a Nucleospin RNA II kit following the instructions of the manufacturer (Macherey-Nagel, Dueren, Germany). For all other RNA isolations, tissues were homogenized using Trizol reagent (Sigma) and stainless-steel beads in a Tissue Lyser (Qiagen), followed by chloroform extraction and RNA precipitation with isopropanol in the presence of 20 µg of glycogen (Fermentas). Pellets were washed twice with 75% ice-cold ethanol and dissolved in MilliQ H2O. RNA concentration and purity were measured using a DS-11 spectrophotometer (Denovix). RNA was kept at -80 °C until further analysis.

### RNA-seq and data analysis

For RNA-seq analysis, total RNA quality of the samples was assayed using Agilent 2100 Bioanalyser (Applied Biosystems). Total RNA concentration was estimated by Qubit Fluorometer (Invitrogen). RNA samples (all with an RNA integrity number RIN ≥ 8.60) prepared from P365 APO-SUS (n=4, pooled) and APO-UNSUS (n=4, pooled) mPFC were analyzed by RNA-seq at the Hudson Alpha Institute for Biotechnology (Huntsville, AL, USA). RNA-seq libraries were formed from approximately 850 ng total RNA of each sample. RNA-seq was performed using paired end sequencing on Illumina HiSeq (Illumina), at 100 base pairs, generating over 30 million paired reads. RNAseq FASTQ files were analyzed using GeneSifter software (VizX Labs). Transcript abundance was calculated by estimating the reads per kilobase of exon per million mapped reads (RPKM) and normalization to the number of mapped reads was used for comparison. The mRNAs differentially expressed in the APO-SUS *versus* APO-UNSUS mPFC and meeting two predefined criteria (fold change ≥ 1.2; Likelihood Ratio test corrected p-value < 0.05) were used as input data for analysis with the Ingenuity Pathway Analysis (IPA) software package (Qiagen) to identify overrepresented biological pathways with a focus on 'Canonical Pathways'.

### Quantitative real-time PCR.

For quantitative real-time PCR (qPCR) analysis, RNA samples were treated with DNase I (Fermentas) and cDNA was synthesized using the Revert Aid H-minus first strand cDNA synthesis kit (Thermo Scientific). cDNA was subsequently diluted 1:20 in MilliQ H₂O and stored at -20 °C until qPCR analysis. qPCR samples were pipetted using a robot (Corbett Robotics) and contained 2.0 µL diluted cDNA, 0.8 µL 5 µM forward primer, 0.8 µL 5 µM reverse primer, 5 µL SybrGreen mix (Roche) and 1.8 µL MilliQ H₂O. qPCR was performed with a Rotor Gene 6000 Series (Corbett Life Sciences) using a 3-step paradigm with a fixed gain of 8. Fifty cycling steps of 95, 60, and 72 °C were applied, and fluorescence was acquired after each cycling step. Primers were designed with NCBI Primer-Blast or Primer Express 2.0 and synthesized by Sigma (for primer pair sequences, see Table 8). Melting temperature was used to check whether a single PCR product was generated and the take off and amplification values of the housekeeping genes (*Ywhaz, B-actin, Ppia* and *Gapdh*) were used to determine the normalization factor with GeNorm after which normalized mRNA expression levels were calculated. For analysis of the developmental time course of mRNA expression, P0, P7, P14 and P21 (+/- 1 day) APO-SUS and APO-UNSUS rats were sacrificed by direct decapitation, brains were immediately removed, and mPFCs were freshly dissected, frozen on dry ice and stored at -80 °C for subsequent RNA extraction and qPCR analysis. Furthermore, for the developmental time course analysis, P21 (+/- 1 day), P28 (+/- 1 day), P90 (+/- 1 day) and P365 (+/- 14 days) APO-SUS and APO-UNSUS brains were removed immediately following decapitation, frozen on dry ice and stored at -80 °C for subsequent micropunching, RNA extraction and qPCR analysis.

### Glutathione assay

Levels of the natural antioxidant glutathione were determined by a kinetic glutathione assay (CS0260; Sigma) that measured the level of total glutathione (oxidized and reduced glutathione). The assay was performed according to the instructions of the manufacturer and glutathione levels were normalized by protein content determination through a bicinchoninic acid (BCA) assay (23225; Thermo Fisher).

### Electron microscopy

For electron microscopy, naïve animals of P90 (+/- 1 day) were perfused with 2% PFA/2% glutaraldehyde and perfused brains were removed, postfixed overnight in 2% PFA/2% glutaraldehyde and stored at 4 °C in PBS/0.01% azide until further processing. Sagittal sections of 100 µm were collected using a vibratome (Leica), fixed with 2% osmium tetroxide and contrast was obtained with 5% uranyl acetate. Following ethanol dehydration, sections were embedded in epon resin and ultrathin (70-100 nm) sections were obtained with an ultramicrotome (Leica). Sections were contrasted using lead citrate and 40 non-overlapping 26000x images were obtained in IL or PL subregions of the mPFC. Myelinated axons were counted in all 40 images of IL region and the G-ratio and axon caliber of all myelinated axons perpendicular to the field of view were measured in FIJI. The percentage of mitochondrial surface was calculated using 49 equally (200 µm) spaced crosses superimposed over 20 randomly picked PL images. The percentage of crosses that touched a mitochondrion over the total number of crosses was calculated and the size of each mitochondrion that touched a cross was measured.

### N-acetyl cysteine treatment

APO-SUS and APO-UNSUS rats were treated between P5-P40 (+/- 1 day) with 0.9 grams per liter and from P40-P90 (+/- 1 day) with 2 grams per liter N-acetyl cysteine (A7250; Sigma) in the drinking water. pH was adjusted to 8.6 using 5M NaOH, equivalent to the pH of control drinking water, and N-acetyl cysteine as well as control drinking water was offered in dark drinking bottles to prevent N-acetyl cysteine oxidation by light. Both N-acetyl cysteine laced and control drinking water were refreshed three times per week. Animals treated with N-acetyl cysteine -laced and control drinking water were either sacrificed at P90 (+/- 1 day) for electron microscopy or immunofluorescent analysis, or were subjected to behavioural testing between P60 and P90 (+/- 6 days) and sacrificed at P90 for glutathione assays and qPCR analyses.

### Immunohistochemistrv

For immunohistochemistry, APO-SUS and APO-UNSUS rats of P90 (+/- 1 day) were perfused with 2% paraformaldehyde (PFA). Perfused brains were removed, postfixed overnight and placed in 30% sucrose in PBS for 3-5 days, frozen on dry ice and stored at -80 °C until further processing. Coronal cryosections of 10 µm were collected in a cryostat (Leica) and rehydrated in 1x PBS, 0.1% Triton X-100 or for OLIG2 NG2 immunohistochemistry in 1x PBS, 0.05% Tween-20. For OLIG2-CC1 staining antigen retrieval was performed in a microwave using citric acid-based antigen unmasking solution (Vector). Tissue was blocked in 4% BSA, 0.1% Triton X-100 or 5% NGS/ NDS/NHS, 1% BSA, 1% glycine, 0.1% lysine, 0.4% triton X-100 for 1 hour at room temperature (RT). Primary antibodies were anti-OLIG2 (AB9610, Millipore 1:1000, ab109186, Abcam 1:400 or MABN50, Millipore 1:500), anti-CC1 (OP80, Calbiochem 1:100), anti-NG2 (MAB5384, Millipore 1:200 or AB5320, Millipore 1:100), anti-MRF ABN45, Millipore 1:100) and incubated overnight at 4 °C. Secondary antibodies were 488-, 555- and 568-conjugated anti-rabbit or anti-mouse (IgG and IgG2b, Alexa) or TRITC-conjugated anti-mouse (IGg1, Southern Biotech) and incubated for 1-2 hours at RT. Hoechst (H6024, Sigma 1:1000) was added as a nuclear counterstain. Sections were mounted in Fluoromount (0100-01, Southern Biotech) and 20x images were obtained using an Axioscan (Leica) and analysed with Zen (Blue edition). Regions of interest were drawn and cells were counted manually. OLIG2+ cell counts were obtained in the OLIG2-CC1 images.

### Primary oligodendroglial cell cultures

Mixed glia cultures were obtained from cortex of P1 APO-SUS and APO-UNSUS rats, n=3 replicates per condition. The tissue was homogenized in mixed glia culture medium (GlutaMAX (Invitrogen), 10% fetal bovine serum (Thermo Fisher) 1% Pen-Strep (Thermo Fisher) and 1% non-essential amino acids (Thermo Fisher)) and cells were kept in this medium on 1:10 poly-L ornithine coated T75 flasks at 37 °C 5% CO₂. Medium was refreshed after 7 and 13 days in culture and at day 14 oligodendrocyte precursor cells (OPCs) were purified using a shaking protocol. Mixed glia cultures were shaken at 250 rpm for 1 hour to discard microglia cells, then cultures were shaken again at 250 rpm for 18 hours to purify OPCs. Supernatant containing OPCs was placed on petridishes (Falcon) three times for 5 minutes to eliminate astrocyte contamination. OPCs were then plated onto PLO-coated coverslips in 24-well plates. After 4 hours, medium was changed to differentiation medium for 4 days (DMEM-F12 (Invitrogen), 0.5% B27 (Sigma) 1% Pen-Strep (Thermo Fisher) and 0.05% 40 ng/ml of T3 thyroid hormone (Sigma)) with or without 1µM Cocl2. After 4 days, cultures were stained with homemade O4 antibody for 1 hour and fixed in 2% PFA for 7 minutes. Subsequently OPCs were incubated with anti-SOX10 (1/100, R&D Systems AF2864) and anti-MBP (1/200, Abcam ab7349) primary antibodies for 1 hour at RT, washed with 1x PBS and incubated with secondary antibodies (donkey anti-TRITC IgM 1/100 (Southern Biotech), donkey anti-goat Alexa 488 1/1000, donkey anti-rat Alexa 647 1/750 and Hoechst) for 1 hour at RT, washed and mounted with fluoromount. Images were obtained in an Axioscan and analysed in Zen (Bleu edition) manually.

### Operant attentional set-shifting tests

Upon the start of behavioural training, rats were food restricted and received 5-7 grams of food per 100 grams of rat daily. Rats were pre-exposed once to grain reward pellets (Rodent Tablet [5TUM], 45mg, TestDiet, USA) in the home cage. Operant conditioning chambers (29.5 cm L, 24 cmW25 cm H;Med Associates, Georgia, VT) were situated in light and sound-attenuating cubicles equipped with a ventilation fan. Each chamber was equipped with two 4.8-cm-wide retractable levers, placed 11.7 cm apart and 6 cm from the grid floor. A cue light (28 V, 100 mA) was present above each lever. At the same wall, a reward pellet could be delivered in a magazine between the levers and a house light (28 V, 100 mA) was located on the same wall. The lever presentation and cue light illumination sides were counterbalanced between rats. Pretraining, operant extra-dimensional set-shifting were performed. Rats performed three extra-dimensional set-shifts (note that between extra-dimensional shifts 2 and 3 reversal learning was performed). The number of errors made until a criterion of 10 subsequent correct trials was reached was measured, and the errors were classified as perseverative errors (following the 'old rule'), regressive errors (following the 'old rule' while more than 70% of previous trials were correct) or never-reinforced errors (pressing a lever that was incorrect during both the 'old rule' and during the current rule).

### Statistical analyses

For qPCR, glutathione assay and electron microscopy analysis, statistical significance was calculated using the independent samples T-test and, when appropriate, with Benjamini-Hochberg correction for multiple comparisons in IBM SPSS Statistics 24. Linear regression with two-way ANOVA was used to test statistical significance of the regression between G-ratio and axon caliber. For the NAC treatment experiment, immunohistochemistry and electron microscopy data analysis was performed using one-way ANOVAs and effects were assessed with independent samples T-tests, and analysis of the set-shifting task was performed using multivariate ANOVAs. For all analyses, outliers were discarded beforehand as indicated by the Grubbs outlier test using Graphpad quickcalcs, and the level of significance was set at p=0.05.

### Results

### Glutathione metabolism-related mRNAs were enriched among the differentially expressed genes in APO-SUS versus APO-UNSUS mPFC

RNA-seq was used to investigate the neurobiological basis of mPFC dysfunction in the APO-SUS rat model with SZ-relevant features. Genome-wide mRNA expression profiling of the mPFC of post-natal day (P) 365 APO-SUS and APO-UNSUS rats showed that 858 transcripts were differentially expressed (fold change ≥|1,2|, Likelihood Ratio test corrected p-value p<0.05) with 371 genes upregulated and 487 genes downregulated in APO-SUS compared to APO-UNSUS mPFC. Ingenuity pathway analysis of the differentially expressed genes revealed that the two top-enriched canonical pathways were 'glutathione-mediated detoxification' and 'glutathione redox reactions I' (p=9.24E-10 and ratio=0.258, and p=1.52E-04 and ratio=0.25, respectively, in Benjamini-Hochberg corrected T-test; Table 1; Tables 2 and 3 for the genes involved in these top two pathways).

**Table 1 - Ingenuity pathway analysis results of RNA sequencing data from mPFC of APO-SUS versus APO-UNSUS rats.**

| **Ingenuity Canonical Pathways** | **-log(Benjamini Hochberg-corrected -H p-value)** | **Ratio of genes present in our data over the total number of genes in the canonical pathway** |
|---|---|---|
| Glutathione-mediated Detoxification | 2.69 | 0.258 |
| Glutathione Redox Reactions I | 2.02 | 0.25 |
| Complement System | 1.87 | 0.189 |
| Cytotoxic T Lymphocyte-mediated Apoptosis of Target Cells | 1.6 | 0.188 |
| Calcium-induced T Lymphocyte Apoptosis | 2.56 | 0.167 |

**Table 2 - Genes in Ingenuity pathway analysis canonical pathway 'Glutathione-mediated detoxification'. Transcriptome-wide analysis of APO-SUS and APO-UNSUS mPFC combined with Ingenuity pathway analysis revealed that this pathway represents the most significantly enriched pathway.**

| **Symbol** | **Entrez Gene Name** | **Corrected p-value** |
|---|---|---|
| Gsta1 | glutathione S-transferase alpha 1 | 0.0264 |
| GSTA1 | glutathione S-transferase alpha 1 | 0.00111 |
| Gsta4 | glutathione S-transferase, alpha 4 | 0.00025 |
| GSTM4 | glutathione S-transferase mu 4 | 0.0354 |
| GSTM5 | glutathione S-transferase mu 5 | 0.000000969 |
| Gstt1 | glutathione S-transferase, theta 1 | 0.00713 |
| HPGDS | hematopoietic prostaglandin D synthase | 0.000794 |
| MGST1 | microsomal glutathione S-transferase 1 | 0.00282 |

**Table 3 - Genes in Ingenuity pathway analysis canonical pathway 'Glutathione redox reactions I'. Transcriptome-wide analysis of APO-SUS and APO-UNSUS mPFC combined with Ingenuity pathway analysis revealed that this pathway represents the second-most significantly enriched pathway.**

| **Symbol** | **Entrez Gene Name** | **Corrected p-value** |
|---|---|---|
| GPX3 | glutathione peroxidase 3 | 0.0183 |
| Gsta1 | glutathione S-transferase alpha 1 | 0.0264 |
| GSTA1 | glutathione S-transferase alpha 1 | 0.00111 |
| Gstt1 | glutathione S-transferase, theta 1 | 0.00713 |
| MGST1 | microsomal glutathione S-transferase 1 | 0.00282 |
| PRDX6 | peroxiredoxin 6 | 0.00E+00 |

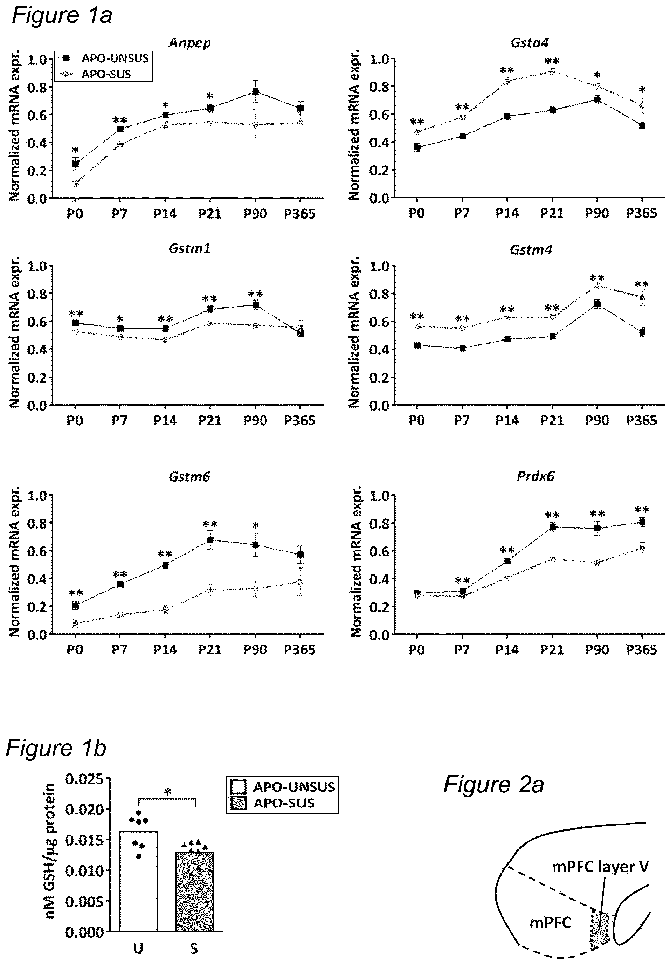

Quantitative real-time PCR (qPCR) analysis confirmed a dysregulation of glutathione-related genes from these two pathways in the mPFC of APO-SUS rats that started early in development (P0) and persisted into late adulthood (P365) (Figure 1a; for exact sample sizes and statistical values in Independent samples T-test with Benjamini-Hochberg multiple comparisons correction, see Table 4). Furthermore, a kinetic assay showed significantly lower levels of the naturally occurring antioxidant tripeptide glutathione in P90 APO-SUS *versus* APO-UNSUS mPFC (Figure 1b; Independent samples T-test t=- 2.817, p=0.015, df=13). Therefore, it is concluded that glutathione antioxidant metabolism was reduced in APO-SUS mPFC throughout postnatal development and adulthood. In addition, the top 35 most significantly differentially expressed genes in APO-SUS mPFC encompassed five downregulated myelin-related mRNAs, in line with previous observation of mPFC hypomyelination in APO-SUS rats; note that the Ingenuity pathway analysis results did not include the myelin-related genes because these genes do not constitute a canonical pathway.

**Table 4 - Statistical values of Independent samples T-test with Benjamini Hochberg multiple comparisons correction in every age group on qPCR expression data presented in Figure 1.**

| **Age** | **Gene** | **t-value** | **df** | **Corrected p-value** |
|---|---|---|---|---|
| P0 | gstm4 | 4.841 | 19 | 0.000* |
| | gsta4 | 3.652 | 18 | 0.002* |
| | prdx6 | -1.402 | 19 | 0.177 |
| | gstm6 | -3.468 | 19 | 0.003* |
| | anpep | -3.070 | 8.378 | 0.015* |
| | gstm1 | -3.184 | 19 | 0.005* |
| P7 | gstm4 | 5.494 | 15.065 | 0.000* |
| | gsta4 | 7.191 | 20 | 0.000* |
| | prdx6 | -3.100 | 20 | 0.006* |
| | gstm6 | -8.665 | 21 | 0.000* |
| | anpep | -3.979 | 20 | 0.001* |
| | gstm1 | -2.296 | 20 | 0.033* |
| P14 | gstm4 | 10.977 | 22 | 0.000* |
| | gsta4 | 8.099 | 20 | 0.000* |
| | prdx6 | -8.349 | 22 | 0.000* |
| | gstm6 | -8.912 | 22 | 0.000* |
| | anpep | -2.374 | 22 | 0.027* |
| | gstm1 | -4.385 | 22 | 0.000* |
| P21 | gstm4 | 6.433 | 20 | 0.000* |
| | gsta4 | 8.913 | 18 | 0.000* |
| | prdx6 | -6.683 | 16.914 | 0.000* |
| | gstm6 | -4.280 | 20 | 0.000* |
| | anpep | -2.784 | 19 | 0.012* |
| | gstm1 | -3.829 | 16.795 | 0.001* |
| P90 | gstm4 | 4.446 | 5.128 | 0.006* |
| | gsta4 | 2.526 | 10 | 0.030* |
| | prdx6 | -3.513 | 10 | 0.006* |
| | gstm6 | -3.111 | 10 | 0.011* |
| | anpep | -1.783 | 10 | 0.105 |
| | gstm1 | -3.760 | 10 | 0.004* |
| P365 | gstm4 | 3.921 | 14 | 0.002* |
| | gsta4 | 2.399 | 14 | 0.031* |
| | prdx6 | -3.719 | 14 | 0.002* |
| | gstm6 | -1.673 | 14 | 0.117 |
| | anpep | -1.170 | 14 | 0.261 |
| | gstm1 | 0.684 | 14 | 0.505 |

| | | | | |
|---|---|---|---|---|
| * Significant after Benjamini Hochberg multiple comparisons correction | | | | |

### Increased number of mitochondria in the mPFC of APO-SUS versus APO-UNSUS rats

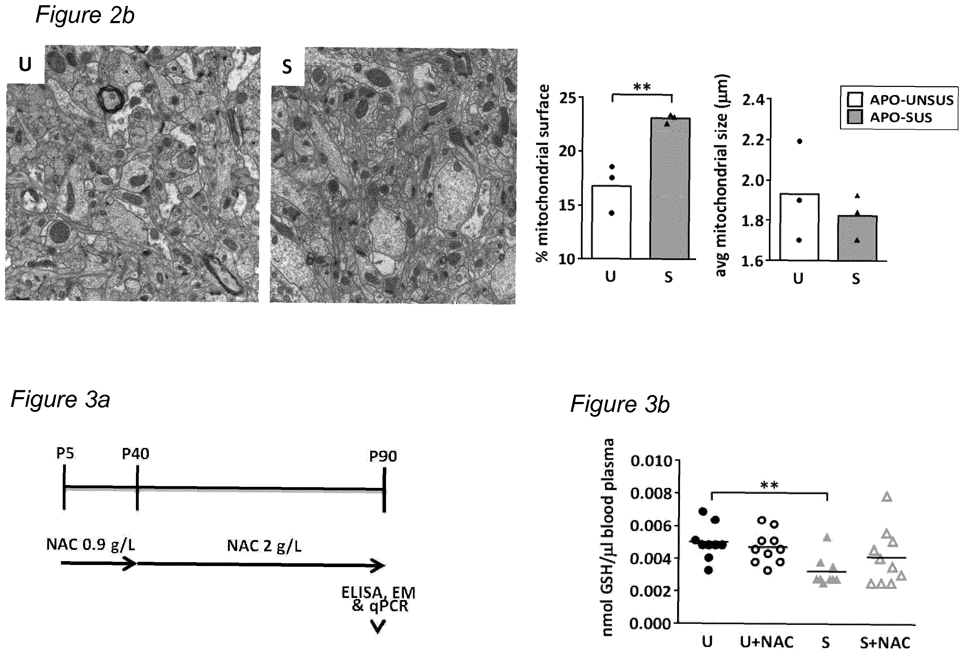

Mitochondria play a key role in the formation of oxidative stress. In SZ, oxidative stress is associated with alterations in mitochondria. To investigate whether the decreased glutathione levels observed in APO-SUS mPFC were accompanied by ultrastructural changes in the mitochondria or by altered mitochondria numbers, transmission electron microscopy analysis was performed. An increased surface area containing mitochondria in APO-SUS was found as compared to APO-UNSUS mPFC (Figure 2a-b; Independent samples T-test t=4.809, p=0.009, df=4). This was true for both axonal and non-axonal mitochondria indicating that the increase in mitochondrial surface in APO-SUS mPFC was not specific to neuronal or non-neuronal cells (Figure 2b; Independent samples T-test axonal mitochondrial surface: t=4.235, p=0.013, df=4, non-axonal mitochondrial surface: t=3.543, p=0.024, df=4). Mitochondrial sizes were not different in APO-SUS *versus* APO-UNSUS mPFC (Figure 2b; Independent samples T-test t=-0.687, p=0.530, df=4). It is therefore concluded that the APO-SUS mPFC contains an increased number of mitochondria compared to APO-UNSUS mPFC.

### NAC treatment restored glutathione metabolism and mitochondria numbers in APO-SUS mPFC

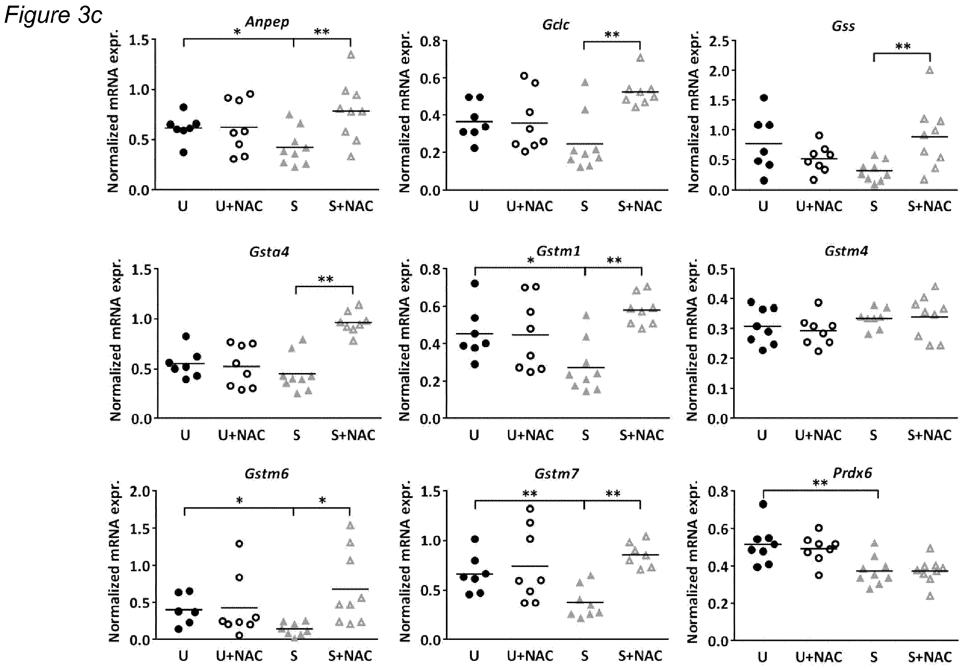

Having established impairment in glutathione metabolism and an increased number of mitochondria in the APO-SUS mPFC, as a next step it was investigated whether treatment of the rats with the glutathione precursor NAC during post-natal development could restore these aberrations in adulthood. APO-SUS and APO-UNSUS rats were treated with NAC from P5 onwards and sacrificed at P90 for molecular and ultrastructural analyses (Figure 3a). A glutathione assay revealed that in APO-SUS mPFC NAC treatment restored glutathione levels to untreated APO-UNSUS levels, while the treatment had no effect on glutathione levels in APO-UNSUS mPFC (Figure 3b; One-way ANOVA F=3.635, p=0.022, df=3, Independent samples T-test APO-SUS *versus* APO-UNSUS t=-3.777, p=0.002, df=16; APO-SUS *versus* APO-SUS+NAC t=-1.406, p=0.178, df=17). Moreover, NAC treatment significantly restored mRNA expression of a number of glutathione-related genes in the mPFC of APO-SUS rats to APO-UNSUS levels, while glutathione-related gene expression remained unaffected in the mPFC of NAC-treated APO-UNSUS rats (Figure 3c; Multivariate ANOVA F=4.997, p<0.001, df=24, for exact statistical values and sample sizes in Independent samples T-test with Benjamini-Hochberg multiple comparisons correction, see Table 6).

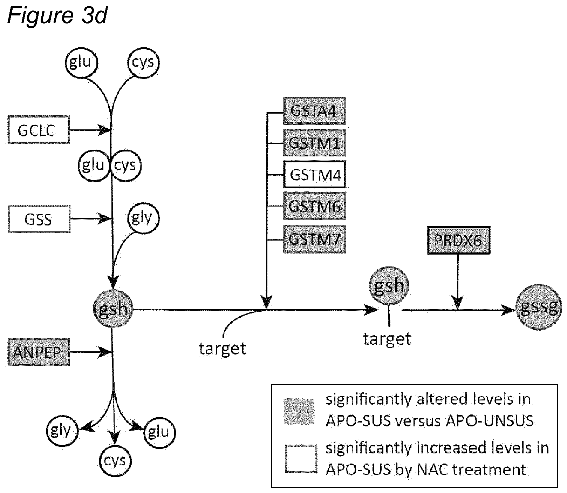

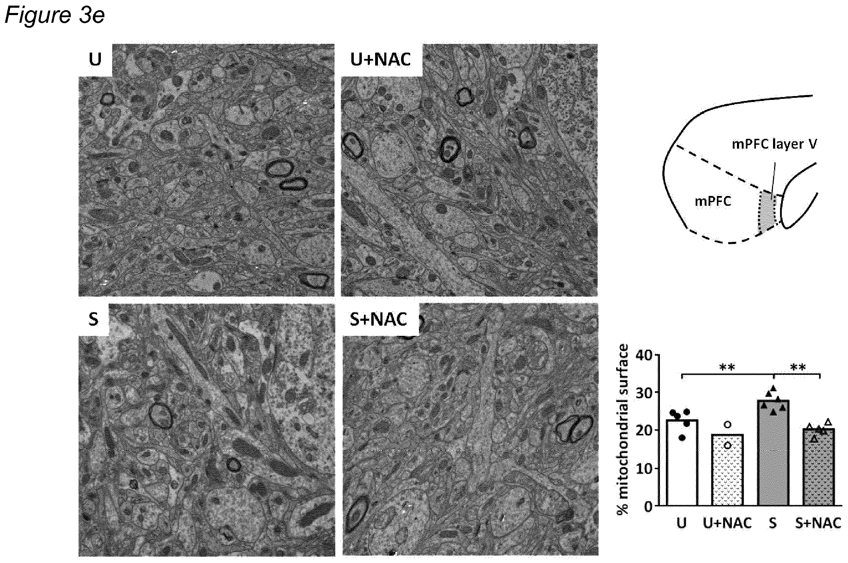

**Table 6 - Statistical values of Independent samples T-test with Benjamini Hochberg multiple comparisons correction on qPCR expression data presented in Figure 3.**

| **APO-SUS versus APO-UNSUS** | | | | **APO-SUS versus APO-SUS+NAC** | | |
|---|---|---|---|---|---|---|
| Gene | **t-value** | **df** | **p-value** | **t-value** | **df** | **corrected p-value** |
| Prdx6 | -3.157 | 15 | 0.007* | 0.021 | 16 | 0.983 |
| Gstm4 | 1.115 | 10.736 | 0.289 | -0.197 | 11.573 | 0.847 |
| Anpep | -2.362 | 14 | 0.033* | -3.070 | 16 | 0.007* |
| Gstm6 | -2.837 | 6.303 | 0.028* | -3.139 | 8.533 | 0.013* |
| Gsta4 | -1.190 | 14 | 0.254 | -6.897 | 15 | 0.000* |
| Gstm1 | -2.568 | 14 | 0.022* | -5.476 | 15 | 0.000* |
| Gclc | -1.782 | 14 | 0.096 | -4.570 | 15 | 0.000* |
| Gss | -2.415 | 7.096 | 0.046* | -3.013 | 9.440 | 0.014* |
| Gstm7 | -3.175 | 13 | 0.007* | -6.484 | 13 | 0.000* |

Interestingly, NAC-treatment increased the mRNA expression of genes that are involved in the production and breakdown of glutathione, as well as the binding of glutathione to target oxidative molecules (Figure 3d). *Gstm4* mRNA expression levels were not influenced by NAC-treatment. It is at present unclear why the expression of Gstm4, with a function similar to that of Gstm1, Gstm6, Gstm7 and Gsta4, is not affected by NAC treatment. The fact that NAC treatment, and thus a change in glutathione levels, did not influence mRNA expression levels of Prdx6 may be linked to its glutathione-independent antioxidant actions, namely through lysophosphatidylcholine acyl transferase and acidic calcium-independent phospholipase A2-dependent activities, in contrast to the solely glutathione-dependent actions of the Gstm and Gsta proteins. Furthermore, ultrastructural analysis showed that the mitochondrial surface in the mPFC was significantly restored to control levels in NAC-treated APO-SUS rats (Figure 3e; One-way ANOVA F=11.360, p<0.001, df=3. Independent samples T-test APO-SUS *versus* APO-UNSUS t=3.288, p=0.009, df=9; APO-SUS *versus* APO-SUS+NAC t=5.972, p<0.001, df=9). It is concluded that NAC treatment during post-natal development alleviates glutathione metabolism and mitochondrial abnormalities in the APO-SUS mPFC.

### NAC treatment restored interneuron hypomyelination in APO-SUS mPFC

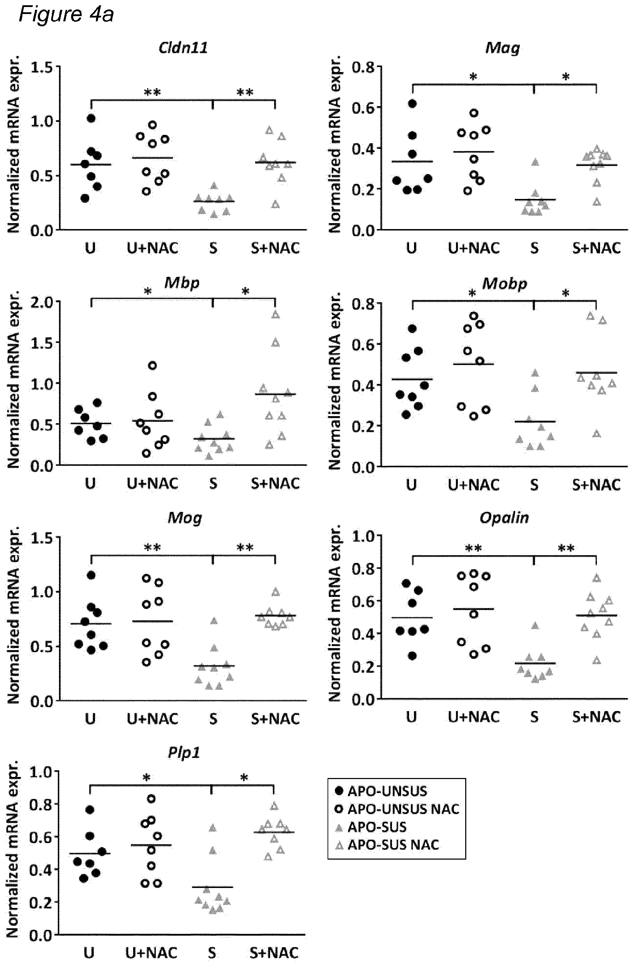

Decreased levels of glutathione are associated with decreased white-matter integrity in the PFC of SZ patients that is thought to result from deficient myelination. In the APO-SUS rats, interneurons are hypomyelinated during adolescent mPFC development. The maturation of interneurons occurs during adolescent mPFC development and is thought to be affected by oxidative stress in SZ. However, it remains unclear whether redox imbalance is a causative factor that induces interneuron hypomyelination in the SZ PFC. To investigate whether decreased levels of glutathione antioxidant in APO-SUS mPFC were indeed the cause of the interneuron hypomyelination, it was tested whether normalizing glutathione metabolism during post-natal development with NAC treatment could rescue interneuron hypomyelination. Strikingly, qPCR analysis revealed a rescue of the mPFC mRNA expression levels of the myelin-related genes examined in APO-SUS rats treated with NAC, while mPFC myelin-related mRNA expression was unaltered in NAC-treated APO-UNSUS rats (Figure 4a; Multivariate ANOVA F=1.864, p=0.037, df=21, for exact statistical values in Independent samples T-test with Benjamini-Hochberg multiple comparisons correction, see Table 7).

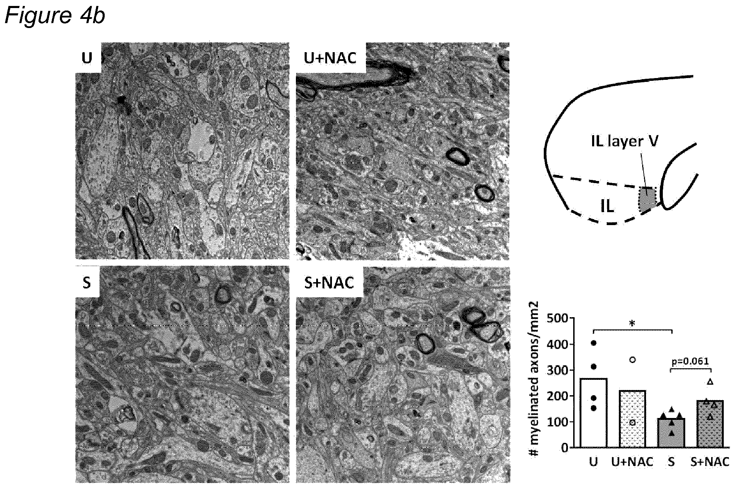

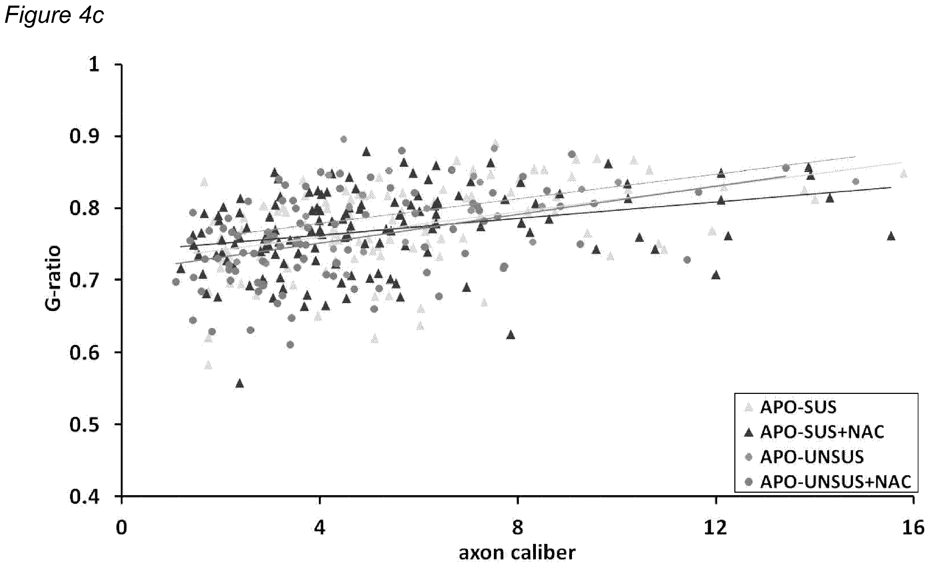

**Table 7 - Statistical values of Independent samples T-test with Benjamini Hochberg multiple comparisons correction on qPCR expression data presented in Figure 4.**

| **APO-SUS versus APO-UNSUS** | | | | **APO-SUS versus APO-SUS+NAC** | | |
|---|---|---|---|---|---|---|
| Gene | **t-value** | **df** | **p-value** | **t-value** | **df** | **corrected p-value** |
| Mog | -3.719 | 15 | 0.002* | -6.022 | 15 | 0.002* |
| Plp | -2.495 | 14 | 0.026* | -4.769 | 15 | 0.026* |
| Mag | -2.901 | 13 | 0.012* | -4.253 | 15 | 0.012* |
| Mobp | -2.929 | 14 | 0.011* | -2.942 | 14 | 0.011* |
| Cldn11 | -3.740 | 13 | 0.002* | -4.424 | 14 | 0.002* |
| Mbp | -2.162 | 14 | 0.048* | -3.010 | 16 | 0.048* |
| Opalin | -4.025 | 13 | 0.001* | -4.667 | 15 | 0.001* |

**Table 8 - Primer sequences.**

| **Gene and abbreviation** | | **Forward primer 5'-3'** | **Reverse primer 5'-3'** |
|---|---|---|---|
| Housekeeping genes | | | |
| β-actin | Beta-actin | CCTTCCTGGGTATGGAA TCCTGT | TAGAGCCACCAATCCAC ACA |
| *Ppia* | Peptidyl-prolyl cis-trans isomerase A | AGCACTGGGGAGAAAGG ATT | AGCCACTCAGTCTTGGC AGT |
| *Gapdh* | Glyceraldehyde-3-phosphate | GGGTGTGAACCACGAGA AAT | ACTGTGGTCATGAGCC CTTC |
| *Ywhaz* | Tyrosine 3-monooxygenase/tryptophan 5-monooxygenase activation protein, zeta | TTGAGCAGAAGACGGAA GGT | GAAGCATTGGGGATCA AGAA |

| Myelin-related genes | | | |
|---|---|---|---|
| *Cldn11* | Claudin 11 | CGCAAAATGGACGAACT GGG | TGCACGTAACCAGGGA GGAT |
| *Mag* | Myelin-associated glycoprotein | AAGCCAGACCATCCAAC CTTC | CTCCTGATTCCGCTCCA AGT |
| *Mbp* | Myelin basic protein | CCCTACTCCATCCTCAG ACTTTCTT | TGGCGGTGTGCCTGTC TAT |
| *Mobp* | Myelin-associated oligodendrocytebasic protein | AATACCTGCAGGGCAAC AAAG | TCTGGTTCTTGGAGAGC CTGG |
| *Mog* | Myelin oligodendrocyte glycoprotein | CGCCGTGGAGTTGAAAG TAG | GCACGGAGTTTTCCTCT AGT |
| *Opalin* | Oligodendrocytic Myelin Paranodal And Inner Loop Protein | ACCCTGATCCAGCGAAG AAG | TGACTGCCTAGGATTCT CGGATA |
| *Plp1* | Proteolipid protein 1 | GGGCCTGAGCGCAACG GTAA | CAGGCACAGCAGAGCA GGCAA |

| Glutathione-related genes | | | |
|---|---|---|---|
| *Gsta4* | Glutathione-S-transferase alpha4 | GCCGCCAAGTACAACTT GTA | CACTGCTAAAGCTAGGC TCTCTTCTT |
| *Gstm4* | Glutathione-S-transferase mu 4 | GCCTAGGCCCCTGGTTT TC | TCTTCACAGCAGCACAG CAACT |
| *Prdx6* | Peroxiredoxin 6 | TGACTGGAAGAAGGGAG AGAGTGT | ATGGGAGCTCTTTGGTG AAGAC |
| *Gclc* | Glutamate-cysteine ligase catalytic subunit | AGAGGACAAACCCCAAC CAC | TCGTGCAAAGAGCCTG ATGT |
| *Gss* | Glutathione synthetase | AGCGTGCCATAGAGAAC GAG | GCTTCCCAGTTCTGTGC GTT |
| *Gstm1* | Glutathione-S-transferase mu 1 | GTCATGCCCACATAGTC TTCATTC | AGTTCAGGGCAGACCT CAAATC |
| *Gstm6* | Glutathione-S-transferase mu 6 | CTGAGCGTTGCTATCTC GGAG | TTCTGTGTATTCCAGGA GCAGC |
| *Anpep* | Alanyl Aminopeptidase | CCCATCAGTGGTTTGGC AAC | CATAGTCAGCACCCAGA AATTCC |

Electron microscopy confirmed that NAC treatment restored the number of myelinated axons in APO-SUS infralimibic (IL) mPFC to APO-UNSUS levels (Figure 4b; One-way ANOVA F=2.419, p=0.121, df=3, Independent samples T-test APO-SUS *versus* APO-UNSUS t=-2.867, p=0.024, df=7; APO-SUS *versus* APO-SUS+NAC t=-2.226, p=0.061, df=7). Furthermore, myelin thickness as measured by the G-ratio remained unaffected, and a significant correlation between G-ratio and axon caliber was found in mPFC of APO-SUS and APO-UNSUS rats with and without NAC treatment (Figure 4c; linear regression APO-UNSUS F=5.495, p=0.029, APO-UNSUS+NAC F=18.637, p<0.001 APO-SUS F=26.548, p<0.001, APO-SUS+NAC F=14.398, p<0.001). Therefore, NAC treatment rescued the hypomyelination phenotype in mPFC of APO-SUS rats without affecting myelin structure.

### NAC treatment restored the number of oligodendrocyte (OL) lineage cells and premyelinating OLs in APO-SUS rats

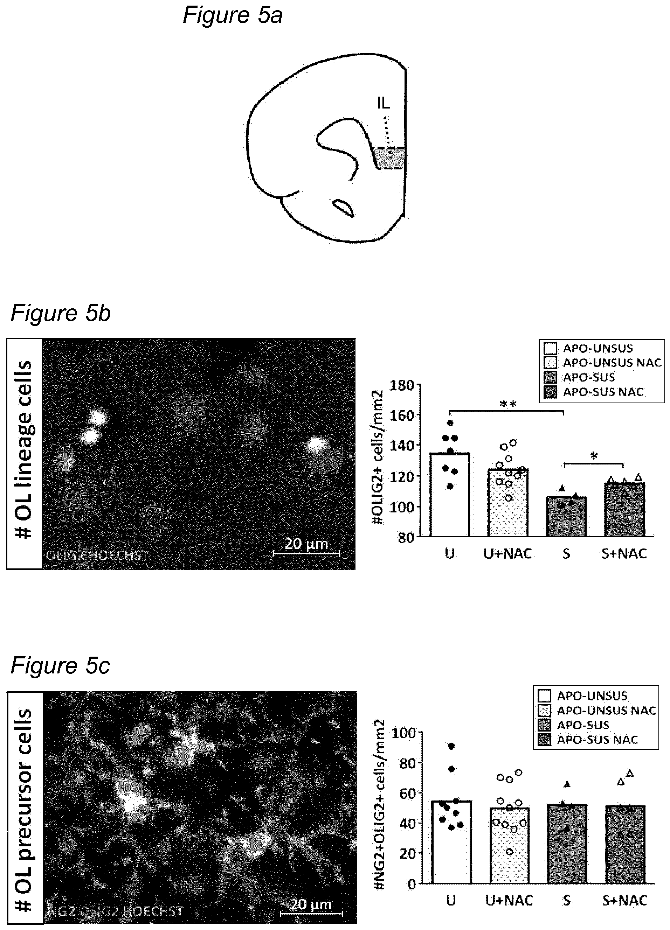

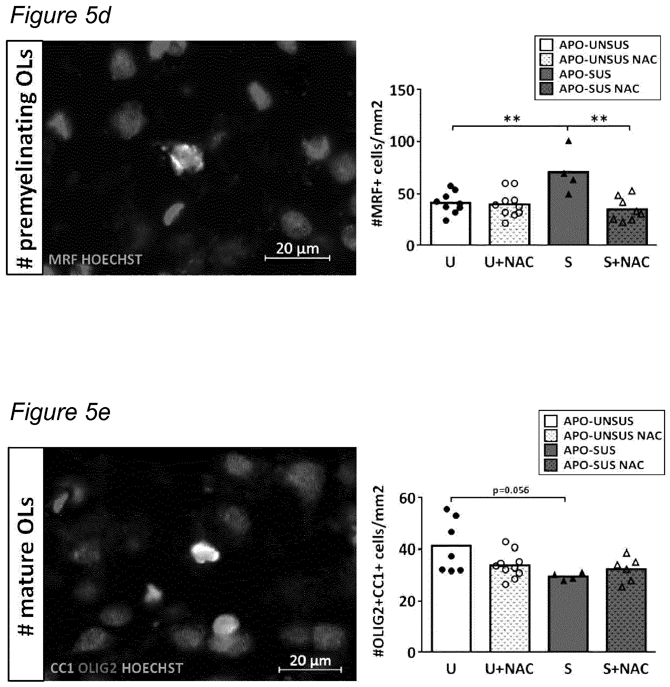

Interneuron hypomyelination in APO-SUS mPFC is caused by impaired maturation of OLs. OLs and in particular premyelinating OLs are highly sensitive to oxidative stress. Importantly, the expression of glutathione-related mRNAs is dysregulated in APO-SUS mPFC from P0 onwards, and as such precedes the decreased expression of myelin-related mRNAs that becomes apparent only during adolescence in APO-SUS mPFC. As myelination of mPFC interneurons takes place during adolescence, it was hypothesized that prolonged oxidative insult to OLs prior to and during adolescence, hinders proper myelination of interneurons. To test this hypothesis, it was investigated whether NAC treatment would alleviate the OL maturation impairment in APO-SUS mPFC. Immunofluorescent staining revealed that in NAC-treated APO-SUS rats the number of OL lineage cells was significantly increased (Figure 5b; One-way ANOVA F=7.530, p=0.001, df=3, Independent samples T-test APO-SUS *versus* APO-UNSUS t=- 4.725, p=0.001, df=8.021, APO-SUS *versus* APO-SUS+NAC t=-3.316, p=0.011, df=8). The number of OL precursor cells (OPCs) (Figure 5c) and mature OLs remained unaffected (Figure 5e), while the number of premyelinating OLs was significantly restored in NAC-treated APO-SUS rats (Figure 5d; One-way ANOVA F=7.275, p=0.001, df=3, Independent samples T-test APO-SUS *versus* APO-UNSUS t=3.420, p=0.006, df=11, APO-SUS *versus* APO-SUS+NAC t=3.850, p=0.003, df=10). These findings suggest that the promyelinating effects of NAC are at least partially caused by a positive effect on OL maturation.

### Oxidative insult blocked APO-SUS and APO-UNSUS OL maturation in vitro

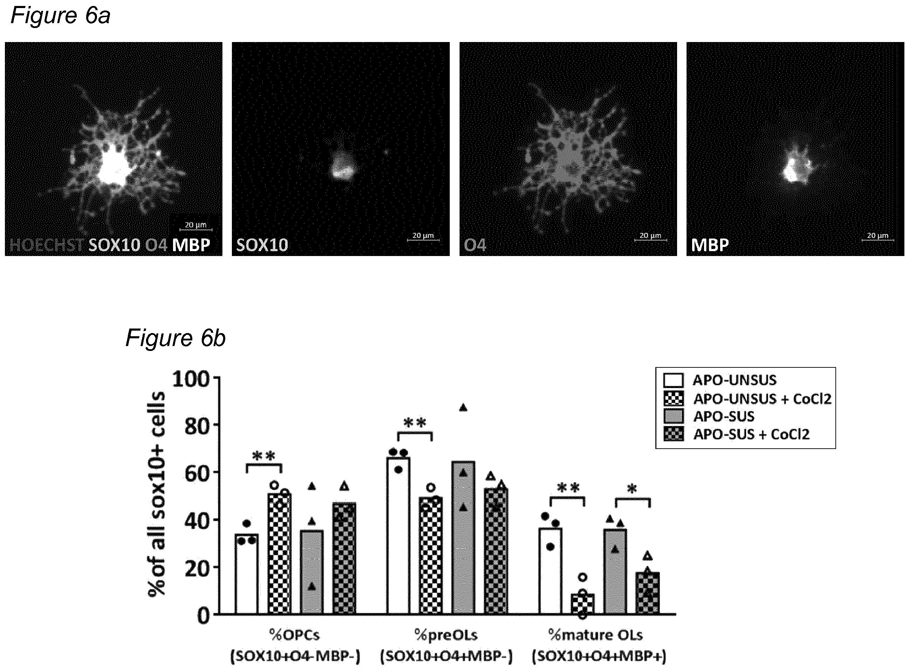

To verify whether an oxidative insult could indeed block the maturation of APO-SUS and APO-UNSUS OLs, *in vitro* experiments were performed with primary OPCs isolated from cortex of newborn APO-SUS and APO-UNSUS rats. OPCs were differentiated in the presence or absence of 1µM cobalt chloride hexahydrate (CoCl₂), a chemical that causes oxidative stress, and the percentages of all SOX10+ oligodendroglial cells that were OPCs (SOX10+O4-MBP-), preOLs (SOX10+O4+MBP-) or mature OLs (SOX10+O4+MBP+) were analyzed. It was confirmed that no differences occur in the intrinsic capacity of APO-SUS and APO-UNSUS OPCs to mature *in vitro.* However, CoCl₂ significantly reduced the percentage of mature OLs in differentiated APO-SUS and APO-UNSUS OPC cultures (Figure 6b; One way ANOVA F=10.436, p=0.004, df=3, Independent samples T-test APO-SUS *versus* APO-SUS+Cocl2 t=2.972, p=0.041, df=4, APO-UNSUS *versus* APO-UNSUS+CoCl₂ t=4.646, p=0.010, df=4). In addition, there was an increase in the percentage of OPCs and a decrease in the percentage of preOLs in APO-UNSUS OPC cultures exposed to CoCl₂ (Figure 6b; Independent samples T-test APO-UNSUS *versus* APO-UNSUS+Cocl2 OPCs t=-4.897, p=0.008, df=4, preOLs t=- 4.897, p=0.008, df=4). It is therefore concluded that oxidative stress indeed hinders OL maturation in OPCs of both APO-SUS and APO-UNSUS rats.

### NAC treatment improved mPFC-dependent cognitive inflexibility in APO-SUS rats

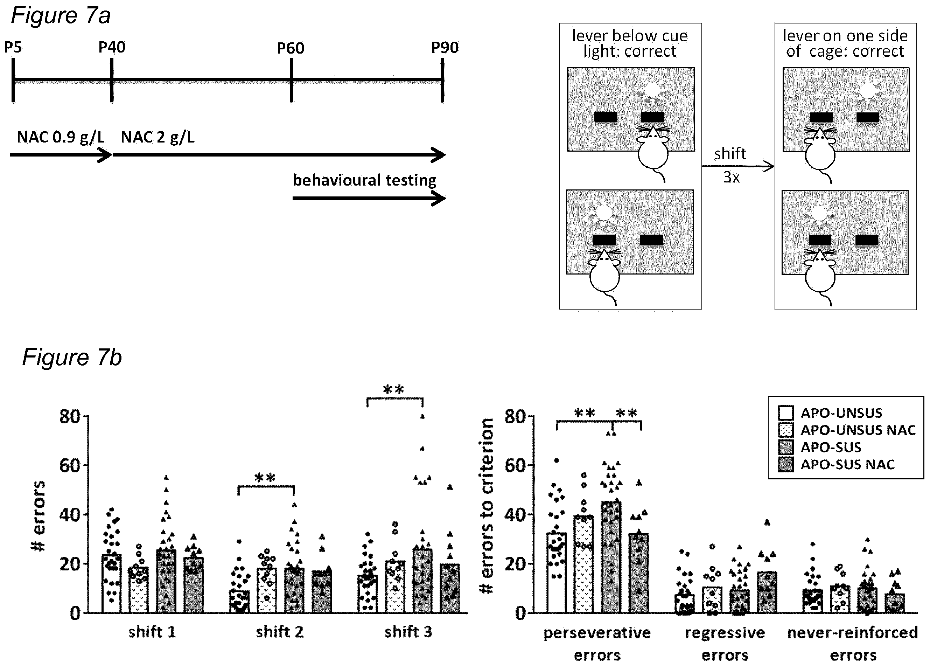

Lastly, it was assessed whether restoring glutathione metabolism, mitochondria numbers, myelin-related gene expression, the number of myelinated axons, OL lineage cells and premyelinating OLs in the APO-SUS mPFC would lead to an improvement of cognitive behaviour in NAC-treated APO-SUS rats. Using the extra-dimensional set-shifting task, mPFC-dependent cognitive inflexibility in APO-SUS rats had been observed. The set-shifting task is modelled after the Wisconsin Card Sorting Test used to examine cognitive symptoms in SZ patients. The extra-dimensional set-shifting task was performed with APO-SUS and APO-UNSUS rats with and without NAC treatment. No differences in initial learning phases of the task were found. In extra-dimensional shift 2 and shift 3, APO-SUS rats showed an increased number of errors until a criterion of a streak of ten correct trials was reached (Multivariate ANOVA F=3.654, p<0.001, df=9 posthoc testing with Benjamini-Hochberg multiple comparisons correction APO-SUS *versus* APO-UNSUS: shift 1 p=0.548, shift 2 p<0.001, shift 3 p=0.008; APO-SUS *versus* APO-SUS+NAC: shift 1 p=0.470, shift 2 p=0.702, shift 3 p=0.272). The number of perseverative errors was significantly increased in untreated APO-SUS rats *versus* untreated APO-UNSUS rats and significantly normalized in APO-SUS rats by NAC treatment (Figure 7b; Multivariate ANOVA F=3.124, p=0.002, df=9 posthoc testing with Benjamini-Hochberg multiple comparisons correction APO-SUS *versus* APO-UNSUS: perseverative errors p<0.001, regressive errors p=0.425, never-reinforced errors p=0.648; APO-SUS *versus* APO-SUS+NAC: perseverative errors p=0.009, regressive errors p=0.012, never-reinforced errors p=0.338).

Taken together, the present data show that NAC treatment strengthens glutathione antioxidant defenses, normalizes the number of mitochondria, improves OL maturation and as such rescues interneuron hypomyelination and improves cognitive inflexibility in the APO-SUS rat model SZ-relevant features.

### Discussion

Animal models recapitulating (some of) the positive, negative and cognitive symptoms of SZ are instrumental to understand the molecular and cellular mechanisms underlying these symptoms. In this study, the well-characterized APO-SUS rat model with SZ-relevant features and an unbiased transcriptomics approach to explore the elusive neurobiological basis of PFC-related cognitive symptoms in SZ were used. Among the genes differentially expressed in mPFC of APO-SUS *versus* APO-UNSUS rats, genes related to glutathione metabolism were enriched. Glutathione is an endogenous antioxidant that decreases oxidative stress. In SZ patients, glutathione levels are reduced in blood plasma, cerebrospinal fluid, PFC and *post-mortem* brain regions, and are independent of disease stage or medication use. The fact that in APO-SUS rats impaired glutathione metabolism was observed already at birth and persisted into late adulthood suggests that oxidative stress in SZ may occur before the clinical symptoms of the disorder become apparent.

The reduced levels of glutathione in APO-SUS mPFC were accompanied by an increase in the number of mitochondria. Mitochondrial dysfunction has been reported in various SZ brain regions, and the interplay between mitochondrial dysfunction and oxidative stress has been hypothesized to play a role in SZ etiology. In individuals at high risk to develop SZ, mitochondrial dysfunction has been linked to symptoms of the disorder, indicating that mitochondrial abnormalities are present already before disease onset. As genetic risk for SZ includes genetic variations in both mitochondria-and oxidative stress-related genes, it is unclear whether mitochondrial dysfunction arises as a consequence of oxidative stress or induces oxidative stress.
Reduced PFC glutathione levels have been associated with decreased PFC white-matter integrity that is thought to arise from defective myelination. Expression of myelin-related mRNAs and proteins is decreased in *post-mortem* SZ frontal cortex and myelin content is reduced in frontal areas of SZ patients. The inventors have recently found that during mPFC development in adolescent APO-SUS rats hypomyelination occurs specifically in interneurons. Notably, during adolescent mPFC development interneurons undergo maturational changes that include myelination and the formation of perineuronal nets, both of which are thought to be affected in SZ by oxidative stress. To investigate whether oxidative stress causes interneuron hypomyelination in the APO-SUS mPFC, APO-SUS rats were treated with the antioxidant glutathione precursor NAC. Chronic post-natal treatment of APO-SUS rats with NAC normalized blood levels of glutathione as well as the levels of mPFC mRNA expression of glutathione metabolism-related genes in adulthood. In addition, NAC treatment significantly reduced the number of mitochondria in APO-SUS mPFC to the number in APO-UNSUS mPFC, indicating that oxidative stress boosts mitochondria numbers. In the mPFC of APO-UNSUS rats, NAC treatment did not affect glutathione levels, glutathione-related mRNA expression nor mitochondria numbers, suggesting that the antioxidant does not influence the redox when it is already in balance. Remarkably, besides its antioxidative properties, NAC also had promyelinating effects in the APO-SUS mPFC. Restoring the redox balance led to a rescue of the number of myelinated axons and myelin-related mRNA expression in the APO-SUS mPFC. Thus, glutathione metabolism appears to be essential for developmental interneuron myelination in SZ. The notion that oxidative stress underlies PFC hypomyelination in SZ is corroborated by the results of analysis of the developmental time course of APO-SUS mPFC mRNA expression which show that the dysregulation of glutathione-related genes is evident already at birth and as such preceded reduced expression of myelin-related genes that starts during adolescence.

Oxidative stress has detrimental effects on cellular proliferation and differentiation, and relative to other cell types OLs, and in particular premyelinating OLs, are extremely vulnerable to oxidative stress. It was therefore hypothesized that the causative role of oxidative stress on interneuron hypomyelination in SZ may be mediated by adverse effects on OL maturation. The inventors have recently shown that OL maturation is impaired in APO-SUS mPFC. Strikingly, it has now been found that treatment with NAC restores the number of OL lineage cells and premyelinating OLs in APO-SUS mPFC to APO-UNSUS levels. Moreover, *in vitro* experiments with APO-SUS and APO-UNSUS OPCs confirmed that an oxidative insult is capable of impairing OPC maturation. The fact that NAC treatment positively influences OL maturation supports the notion that oxidative damage to OLs contributes to interneuron hypomyelination in the APO-SUS mPFC and may well underlie hypomyelination in SZ.

Intriguingly, the antioxidative and promyelinating effects of NAC in APO-SUS mPFC led to a significant improvement in cognitive inflexibility in the extra-dimensional set-shifting task. This task is equivalent to the Wisconsin Card Sorting Test that is used to reveal cognitive inflexibility in SZ patients. As an add-on treatment to antipsychotic medication, NAC has moderate effects on the positive, negative and cognitive symptoms of chronic SZ patients. The effects on cognition were replicated in early-psychosis patients and accompanied by increases in PFC glutathione levels. However, treatment with N-acetylcysteine amide (NACA), a more hydrophobic and lipophilic derivative of NAC allowing higher ability to cross the blood brain barrier, might boost the effects seen in SZ patients treated with NAC. The myelination process can also be accelerated by behavioural experiences, such as physical exercise, social interactions and environmental enrichment. Behavioural therapy is known to enhance white-matter integrity and improve cognition in SZ patients. The inventors have recently found that chronic environmental enrichment during adolescence can indeed rescue interneuron hypomyelination as well as cognitive inflexibility in the APO-SUS rats.

Collectively, the findings increase the understanding of the neurobiological sequence of events leading to cognitive symptoms in SZ and provide new avenues for the development of treatment strategies for this devastating neurodevelopmental psychiatric disorder. The present studies support the use of chronic NAC (or NACA) treatment in combination with PFC-directed behavioural therapy as preventative measures for individuals at high risk for developing SZ and early-phase SZ patients.

### Description of the drawings

The present invention will be discussed in more detail below, with reference to the attached drawings, in which:
Figure 1: Decreased glutathione-related mRNA expression and glutathione levels in APO-SUS versus APO-UNSUS mPFC. (a) Developmental timeline of mRNA expression of the glutathione-related genes alanyl aminopeptidase-N (Anpep), glutathione-s-transferase-α4 (Gsta4), glutathione-s-transferase- µ1 (Gstm1), glutathione-s-transferase-µ4 (Gstm4), glutathione-s-transferase-µ6 (Gstm6) and peroxiredoxin 6 (Prdx6) in APO-SUS and APO-UNSUS mPFC. (b) nanoMol GSH per microgram protein in P90 APO-SUS versus APO-UNSUS mPFC. *p<0.05 and **p<0.01 in independent samples T-test with Benjamini Hochberg multiple comparisons correction. Error bars represent standard error of the mean.
Figure 2: Increased mitochondrial surface, but no change in mitochondrial size in APO-SUS mPFC. (a) Schematic representation of mPFC adapted from the Paxinos and Watson rat brain atlas. (b) Electron microscopic representative images and analysis of APO-SUS (n=3) and APO-UNSUS (n=3) average total, axonal and non-axonal mitochondrial surface areas and mitochondrial size in 20 randomly-picked images per animal.
Figure 3: N-acetyl cysteine treatment restored glutathione levels, glutathione-related mRNA expression, and mitochondrial surface in APO-SUS mPFC. (a) Schematic representation of experimental paradigm. APO-SUS and APO-UNSUS rats were treated with N-acetyl cysteine 0.9 g/L from P5 to P40 and with N-acetyl cysteine 2 g/L from P40-P90 and rats were sacrificed for ELISA, EM or qPCR at P90. (b) Glutathione (GSH) levels per microliter blood plasma in APO-SUS (S n=9, S+NAC n=10) and APO-UNSUS (U n=9, U+NAC n=10) rats with and without N-acetyl cysteine treatment. (c) qPCR analysis of mRNA expression of the glutathione-related genes alanyl aminopeptidase-N (Anpep), glutathione cysteine ligase catalytic subunit (Gclc), glutathione synthethase (Gss), glutathione-s-transferase-a4 (Gsta4), glutathione-s-transferase-µ1 (Gstm1), glutathione-s-transferase-µ4 (Gstm4), glutathione-s-transferase-µ6 (Gstm6), glutathione-s-transferase-µ7 (Gstm7) and peroxiredoxin 6 (Prdx6) in mPFC of APO-SUS and APO-UNSUS rats with and without N-acetyl cysteine treatment (exact sample sizes displayed in graphs). (d) Schematic representation of glutathione (GSH) metabolic pathway. Circles represent amino acids, squares represent enzymes, green symbols refer to components with significantly altered levels in APO-SUS versus APO-UNSUS mPFC, blue outlines refer to components with significantly increased levels in APO-SUS after N-acetyl cysteine treatment (e) Electron microscopic representative images and analysis of the average mitochondrial surface in mPFC of APO-SUS (S n=6, S+NAC n=5) and APO-UNSUS (U n=5, U+NAC n=2) rats with and without N-acetyl cysteine treatment from 20 randomly-picked images per animal. *p<0.05, **p<0.01 in Independent samples T-test with Benjamini-Hochberg multiple comparisons correction.
Figure 4 - N-acetyl cysteine treatment rescued hypomyelination in APO-SUS mPFC. (a) Normalized mRNA expression of myelin-related genes claudin 11 (Cldn11), myelin associated glycoprotein (Mag), myelin basic protein (Mbp), myelin oligodendrocyte basic protein (Mobp), oligodendrocyte glycoprotein (Mog), Oligodendrocytic Myelin Paranodal And Inner Loop Protein (Opalin), proteolipid protein 1 (Plp1) in mPFC of APO-SUS and APO-UNSUS rats with and without N-acetyl cysteine treatment (exact sample sizes displayed in graphs). (b) Electron microscopic representative images and quantification of the number of myelinated axons in APO-SUS and APO-UNSUS rats with and without N-acetyl cysteine treatment. (c) G-ratio versus axon caliber for all myelinated axons in IL of APO-SUS and APO-UNSUS rats with and without N-acetyl cysteine treatment. *p=<0.05 **p=<0.01 in Independent samples T-test. Error bars represent standard error of the mean.
Figure 5: N-acetyl cysteine treatment restored the number of OL lineage cells and the number of premyelinating OLs in APO-SUS IL. (a) Schematic representation of IL in rat brain adapted from the Paxinos and Watson rat brain atlas. Numbers of (b) OL lineage cells (OLIG2+), (c) OL precursor cells (OLIG2+ NG2+), (d) premyelinating OLs (MRF+) and (e) mature OLs (OLIG2+CC1+) per mm2 in the IL of APO-SUS versus APO-UNSUS rats with and without N-acetyl cysteine treatment (n=4-11 specified in the graphs). *p=<0.05 **p=<0.01 in independent samples T-test.
Figure 6: Oxidative stress induced OL maturation impairment in APO-SUS ad APO-UNSUS primary OPCs. (a) Representative images and (b) quantification of the percentages of all SOX10+ oligodendroglia cells that are OPC (SOX10+O4-MBP-), premyelinating OL (preOL) (SOX10+O4+MBP-) and mature OL (SOX10+O4+MBP+) in APO-SUS versus APO-UNSUS primary oligodendroglia cultures treated with and without Cocl2 during 4 days differentiation (n=3). *p=<0.05 **p=<0.01 in independent samples T-test.
Figure 7: N-acetyl cysteine treatment improved cognitive inflexibility in APO-SUS rats. (a) Schematic representation of the experimental design and extra-dimensional set-shifting task. APO-SUS and APO-UNSUS rats were treated with N-acetyl cysteine 0.9 g/L from P5 to P40 and with N-acetyl cysteine 2 g/L from P40-P90. Behavioural testing was performed between P60 and P90. Rats were trained to press the lever above which a cue light was illuminated. From trial 21 of the next session onwards, rats were required to press the lever on one side of the cage irrespective of the cue light. (b) Number of errors to criterion (streak of 10 correct trials) and total number of perseverative, regressive and never-reinforced errors in APO-UNSUS (U n=28, U+NAC n=10) and APO-SUS (S n=30, S+NAC n=10) rats with and without N-acetyl cysteine treatment. **p<0.01 in multivariate ANOVA posthoc testing with Benjamini Hochberg multiple comparisons correction.

## Claims

1. Composition comprising N-acetyl cysteine or a derivative thereof selected from the group of compounds represented by formula (II), pharmaceutically acceptable salts thereof, compounds represented by formula (III) and pharmaceutically acceptable salts thereof: 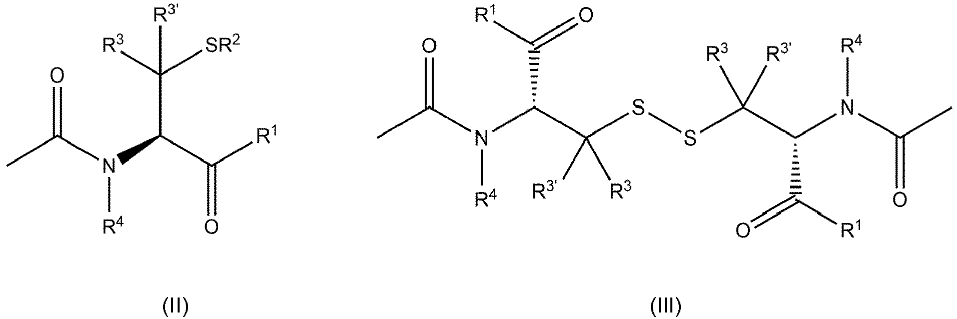 wherein:
-R¹ represents -OH, -NH₂, -NHR⁵ or -OR⁵ wherein -R⁵ represents a straight-chain or branched C₁₋₆-alkyl, a straight-chain or branched C₂₋₆-alkenyl, phenyl or phthalidyl, preferably -R¹ represents -OH, - NH₂ -OCH₃, -OCH₂CH₃, -OCH₂CH₂CH, -OCH(CH₃)CH₃ or -CH₂CHCH₂, more preferably -R¹ represents -OH, -NH₂ -OCH₃ or -OCH₂CH₃, still more preferably -R¹ represents -OH or -NH₂;
-R² represents -H, -R⁶ or -COR⁶, wherein -R⁶ represents a straight-chain or branched C₁₋₆-alkyl, a straight-chain or branched C₂₋₆-alkenyl or phenyl, preferably -R² represents -H, -CH₃, -CH₂CH₃, - COCH₃, -COCH₂CH₃, or -CH₂CHCH₂, more preferably -R² represents -H, -COCH₃ or -CH₃, still more preferably -R² represents -H;
-R³ and -R³' individually represent -H or -CH₃, preferably -R³ and -R³' are the same, more preferably -R³ and -R³' represent -H; and
-R⁴ represents -H, -R⁷ or -COR⁷, wherein -R⁷ represents a straight-chain or branched C₁₋₆-alkyl, a straight-chain or branched C₂₋₆-alkenyl or phenyl, preferably -R⁴ represents -H, -CH₃, -CH₂CH₃, - COCH₃, -COCH₂CH₃ or -CH₂CHCH₂, more preferably -R⁴ represents -H, -COCH₃ or -CH₃, still more preferably -R⁴ represents -H;
for use in a method of treating and/or preventing one or more cognitive symptoms in a subject suffering from schizophrenia or at risk of suffering from schizophrenia.

2. Composition for use according to claim 1, wherein said composition comprises N-acetyl cysteine (NAC) or a pharmaceutically acceptable salt thereof.

3. Composition for use according to claim 1, wherein said composition comprises N-acetyl cysteine amide (NACA) or a pharmaceutically acceptable salt thereof.

4. Composition for use according to any one of the preceding claims, wherein said composition is provided in a unit dosage form comprising the N-acetyl cysteine (derivative) in an amount that is equipotent to about 500-4000 mg of N-acetyl cysteine, more preferably to about 750-3500 mg of N-acetyl cysteine, to about 1000-3000 mg of N-acetyl cysteine, or to about 1200-2500 mg of N-acetyl cysteine.

5. Composition for use according to any one of the preceding claims, wherein said composition comprises a second therapeutic ingredient that has efficacy in the treatment of one or more symptoms associated with schizophrenia, wherein said second therapeutic ingredient is selected from the group of psychotropic agents; the group of agents acting on the glutamatergic system, agents acting on the cholinergic system, agents acting on the serotonergic system, agents acting on the dopaminergic system, agents acting on the GABA-ergic system, agents acting on the adrenergic system and agents acting on the noradrenergic system; the group of agents that increase plasma levels of glutathione; the group of agents that exert antioxidative effects; and the group of agents that has efficacy in the treatment of the positive symptoms associated with schizophrenia.

6. Composition for use according to any one of the preceding claims, wherein said method is directed at the treatment of cognitive inflexibility, at improving cognitive flexibility, at preventing cognitive inflexibility and/or at maintaining cognitive flexibility, in a subject suffering from schizophrenia or at risk of suffering from schizophrenia.

7. Composition for use according to claim 6, wherein said method is directed at the treatment of cognitive inflexibility and/or at improving cognitive flexibility, as reflected by a decrease in the total number of errors (total number of errors and/or perseverative errors) in the WCST during the treatment period; and/or the prevention of cognitive inflexibility and/or at maintaining cognitive flexibility, as reflected by a more or less constant total number of errors (total number of errors and/or perseverative errors) in the WCST during the treatment period.

8. Composition for use according to any one of the preceding claims, wherein said method is directed at the treatment and/or prevention of one or more PFC- dependent cognitive symptoms.

9. Composition for use according to claim 8, wherein said PFC-dependent cognitive symptoms are selected from a deficit in cognitive flexibility, social cognition, processing speed, response to novelty and behavioral-emotional self-regulation, most preferably said one or more PFC-dependent cognitive symptoms is a cognitive flexibility deficit.

10. Composition for use according to any one of the preceding claims, wherein said subject is a human and has an age of 12 years or older.

11. Composition for use according to any one of the preceding claims, wherein said treatment comprises the oral administration of the composition at a daily dose that is equipotent to about 1000-8000 mg of N-acetyl cysteine, more preferably to about 1500-7000 mg of N-acetyl cysteine, to about 2000-6000 mg of N-acetyl cysteine, to about 2400-5000 mg of N-acetyl cysteine.

12. Composition for use according to any one of the preceding claims, wherein the treatment comprises the repeated administration of the composition.

13. Pharmaceutical kit comprising a package containing a plurality of unit dosage forms as defined in claim 4 and a leaflet containing printed instructions to repeatedly self-administer said unit dosage forms by oral ingestion.

14. Method of improving cognitive functioning, especially executive functioning, in a human subject suffering from schizophrenia or at risk of suffering from schizophrenia, said method comprising the administration of a composition comprising N-acetyl cysteine or a pharmaceutically acceptable derivative thereof.

15. Use of N-acetyl cysteine or a pharmaceutically acceptable derivative thereof in the manufacture of a medicament for use in a method of improving cognitive functioning, especially executive functioning, in a human subject suffering from schizophrenia or at risk of suffering from schizophrenia.
